# DEMANDE DE BREVET EUROPEEN

(11) **EP 1 870 088 A2**
(43) Date de publication de la demande: **26.12.2007**
(21) Numéro de dépôt: 07301103.3
(22) Date de dépôt: 12.06.2007
(51) Int. Cl.: A61K 8/55, A61K 8/898, A61K 8/899, A61Q 1/04, A61Q 1/06

(54) **Composition cosmétique pour les lèvres associant un tensioactif phosphaté et un polymère siliconé**

(30) Priorité: 13.06.2006 FR 0652111
(71) Demandeur: L'ORÉAL, 75008 Paris (FR)
(72) Inventeur: Ilekti, Philippe, 94700, MAISON-ALFORT (FR); Le Chaux, Laure, 94240, L'HAY-LES-ROSES (FR)
(74) Mandataire: Le Coupanec, Pascale A.M.P.

(57) **Abrégé**

La présente invention concerne une composition cosmétique pour les lèvres comprenant dans un milieu physiologiquement acceptable au moins :
- un tensioactif phosphaté, et
- un polymère siliconé comportant au moins un motif comprenant :
(1) des polyorganosiloxanes comportant au moins deux groupes capables d'établir des interactions hydrogène, ces deux groupes étant situés dans la chaîne du polymère, et/ou
(2) des polyorganosiloxanes comportant au moins deux groupes capables d'établir des interactions hydrogène, ces deux groupes étant situés sur des greffons ou ramifications.

## Description

La présente invention se rapporte à une composition cosmétique pour les lèvres comprenant au moins un tensioactif phosphaté et un polymère siliconé.

Dans les produits cosmétiques ou dermatologiques, il est courant de trouver une phase liquide structurée, à savoir gélifiée et/ou rigidifiée. Ceci est notamment le cas dans les compositions solides, en particulier les compositions coulées solides, les baumes et les rouges à lèvres, les fards à paupières, les produits anti-cernes et les fonds de teint coulés. Cette structuration est classiquement obtenue à l'aide de cires ou de charges ou plus récemment à partir de gélifiants spécifiques.

Ainsi, dans les documents WO-A-97/36573, US-A-5 874 069, US-A-5 919 441, US-A-6 051 216, WO-A-02/17870, WO-A-02/17871, EP-A-1 177 784, WO-A-99/06473, et US-A-6 353 076 qui est une division du US-A-6 051 216, sont proposées des compositions cosmétiques telles que des sticks ou des gels de déodorant, comprenant une phase huileuse siliconée gélifiée par un polymère siliconé de type polysiloxane/polyamide.

L'utilisation de polymères siliconés permet d'accéder à une texture solide originale de composition cosmétique, à savoir dotée d'une rigidité relativement faible et d'une élasticité élevée. Cette texture ne correspond ni à celle d'un stick conventionnel doté d'une rigidité assez élevée, ni à celle d'un gel classique dont la consistance est liquide ou pâteuse.

Conjointement à cet ajustement de texture, les inventeurs ont cherché à accéder à une formulation de composition qui soit en outre dotée de bonnes qualités de déformabilité pour une application aisée et agréable et qui puisse donner également, si besoin est, satisfaction en terme de brillance.

De manière inattendue, les inventeurs ont constaté que l'utilisation d'un tensioactif phosphaté en association avec un tel polymère siliconé permettait d'obtenir des compositions donnant satisfaction en ces termes.

En conséquence, la présente invention concerne selon un premier aspect, une composition cosmétique pour les lèvres comprenant dans un milieu physiologiquement acceptable au moins :
- un tensioactif phosphaté, et
- un polymère siliconé comportant au moins un motif comprenant :
   (1) des polyorganosiloxanes comportant au moins deux groupes capables d'établir des interactions hydrogène, ces deux groupes étant situés dans la chaîne du polymère, et/ou
   (2) des polyorganosiloxanes comportant au moins deux groupes capables d'établir des interactions hydrogène, ces deux groupes étant situés sur des greffons ou ramifications.

Plus particulièrement, les compositions selon l'invention sont à l'état solide.

L'invention permet d'obtenir des compositions ayant un glissant amélioré et leur application est agréable. Le dépôt sur les lèvres est homogène.

La présente invention a également pour objet un procédé de maquillage des lèvres dans lequel on applique sur les lèvres une composition telle que définie précédemment.

Elle concerne de même selon un autre de ses aspects, l'utilisation d'un polymère siliconé en association avec un tensioactif phosphaté pour préparer une composition cosmétique pour les lèvres, apte à procurer un film de brillance améliorée.

Selon un autre de ses aspects, elle concerne l'utilisation d'un polymère siliconé en association avec un tensioactif phosphaté pour assurer une dispersion homogène et stabilisée de particules solides, telles que par exemple des pigments, dans une composition cosmétique pour les lèvres.

Bien qu'également compatible avec une formulation solide, à l'image d'un stick, ou fluide comme un gloss, les compositions selon l'invention s'avèrent particulièrement utiles pour accéder à une formulation dotée simultanément de propriétés souples et élastiques.

De manière inattendue, les compositions selon l'invention s'avèrent ainsi dotées d'une texture qui peut être associée à une gestuelle d'application particulière.

Par exemple, les textures solides souples et élastiques obtenues selon l'invention peuvent être compatibles avec une application directe, sur les lèvres sans requérir l'utilisation d'applicateur comme dans le cas de textures fluides. Les compositions selon l'invention manifestent en outre lors de leur application, une souplesse, une douceur et une très bonne élasticité préservant ainsi le produit pour une prochaine application. Le produit peut ne pas se déformer de façon définitive et reprendre sa forme initiale après application.

Sous la forme d'un dôme, les compositions conformes à l'invention manifestent avantageusement le comportement d'un solide élastique déformable et souple, conférant à l'application une douceur remarquable.

Outre les avantages précités, l'association considérée de l'invention, à savoir tensioactif phosphaté et polymère siliconé, s'avère particulièrement avantageuse pour disperser de manière homogène des particules et notamment des pigments au sein des compositions cosmétiques les contenant. De plus, les compositions selon l'invention sont également préservées des phénomènes d'exsudation bien entendu indésirables.

### Caractérisation de l'élasticité et de la dureté

Avantageusement, les compositions selon l'invention possèdent une dureté variant de 10 à 250 g et/ou une élasticité supérieure à 80 %.

La dureté et l'élasticité de la composition selon l'invention peuvent être mesurées à l'aide d'un texturomètre qui permet d'obtenir la variation de la résistance à la déformation de la composition en fonction du déplacement d'un mobile dans un échantillon de ladite composition.

Le texturomètre mesure la force de résistance à la déformation de la composition dès que le mobile entre en contact avec l'échantillon. Après avoir atteint une profondeur maximale programmée L0 dans l'échantillon, le mobile retourne au point initial.

La dureté (exprimée en gramme ou en Newton) est égale à la valeur de résistance de la composition lorsque le mobile est en bout de course, et l'élasticité (exprimée en pourcentage) est égale au rapport de i) la distance L à laquelle se produit la rupture de contact entre le mobile et l'échantillon au cours du retrait du mobile et ii) de la distance L0. La rupture de contact se traduit par l'annulation de la force de résistance de la composition sur le mobile.

L'élasticité est proportionnelle à la distance sur laquelle le système va « accompagner » la remontée du mobile ; plus elle est importante, plus le système est élastique.

Le texturomètre utilisé peut notamment être un texturomètre Stable Micro System TAX-T2i^{®} équipé du logiciel d'exploitation type Texture Expert Exceed^{®} muni d'un mobile plastique hémisphérique P/0.5 HS Rheo's.

Les paramètres appliqués sont avantageusement les suivants :
- vitesse avant contact : 0,1 mm.s-1,
- vitesse de déplacement dans l'échantillon : 0,1 mm.s-1,
- vitesse de retrait : 0,1 mm.s-1,
- profondeur maximale L0 1 mm.

On prépare les échantillons de composition en coulant à chaud une quantité suffisante de la composition à tester, par exemple dans une boite de Pétri 100 x 15 mm préalablement tarée, pour obtenir un échantillon d'environ 1 cm d'épaisseur. L'intérêt du choix de ce conditionnement est sa largeur suffisante pour s'affranchir de tout effet de bord. On prépare ainsi deux boîtes de Pétri, qui sont laissées au repos un minimum de 24 h à 20 °C avant caractérisation.

Au minimum trois mesures sont réalisées sur chaque échantillon: une, au centre et d'autres, sur des points situés à équidistance du centre et du bord de celle-ci.

La dureté et l'élasticité sont égales à la moyenne des mesures effectuées, au nombre minimum de six.

La dureté de la composition selon l'invention est telle que la composition est autoportée et peut se déliter aisément pour former un dépôt satisfaisant sur la peau et les lèvres. En outre, avec cette dureté, la composition de l'invention résiste bien aux chocs.

Plus particulièrement, la dureté peut varier de 30 g à 200 g, notamment de 50 g à 190 g, voire de 70 à 175 g et plus particulièrement de 100 g à 150 g.

Les compositions selon l'invention possèdent avantageusement une élasticité supérieure à 90 % et en particulier proche de 95 %.

### Polymère siliconé

Comme indiqué précédemment, les compositions selon l'invention comprennent au moins un polymère siliconé.

Les polymères siliconés de la composition sont de préférence solides à la température ambiante (25 °C) et pression atmosphérique (760 mm de Hg).

Par polymère, on entend au sens de l'invention un composé ayant au moins 2 motifs de répétition, de préférence au moins 3 motifs de répétition et mieux encore 10 motifs de répétition.

Les polymères siliconés de la composition de l'invention sont des polymères du type polyorganosiloxane comme par exemple ceux décrits dans les documents US-A-5 874 069, US-A-5,919,441, US-A-6,051,216 et US-A-5,981,680.

Selon l'invention, les polymères siliconés peuvent appartenir aux deux familles suivantes :
(1) des polyorganosiloxanes comportant au moins deux groupes capables d'établir des interactions hydrogène, ces deux groupes étant situés dans la chaîne du polymère, et/ou
(2) des polyorganosiloxanes comportant au moins deux groupes capables d'établir des interactions hydrogène, ces deux groupes étant situés sur des greffons ou ramifications.

Les groupes capables d'établir des interactions hydrogène peuvent être choisis parmi les groupes ester, amide, sulfonamide, carbamate, thiocarbamate, urée, uréthane, thiourée, oxamido, guanidino, biguanidino et leurs combinaisons.
A) Selon une première variante, les polymères siliconés sont des polyorganosiloxane tels que définis ci-dessus et dont les motifs capables d'établir des interactions hydrogènes sont disposés dans la chaîne du polymère.
   Les polymères siliconés peuvent plus particulièrement être des polymères comprenant au moins un motif répondant à la formule générale I : 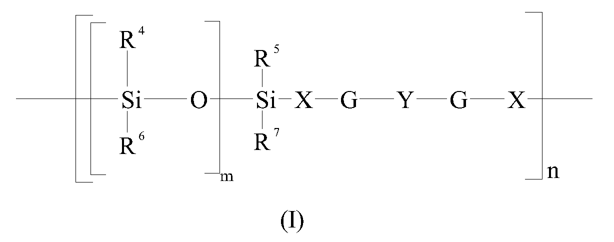 dans laquelle :
   1) R⁴, R⁵, R⁶ et R⁷, identiques ou différents, représentent un groupe choisi parmi :
      - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
      - les groupes aryles en C₆ à C₁₀, éventuellement substitués par un ou plusieurs groupes alkyle en C₁ à C₄,
      - les chaînes polyorganosiloxanes contenant ou non un ou plusieurs atomes d'oxygène, de soufre et/ou d'azote,
   2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
   3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
   4) Y représente un groupe répondant à la formule : 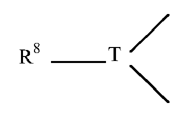 dans laquelle
      - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
      - R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
   5) les G, identiques ou différents, représentent les groupes divalents choisis parmi: 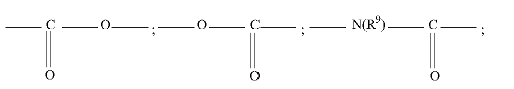 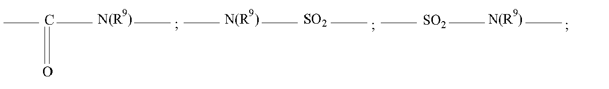 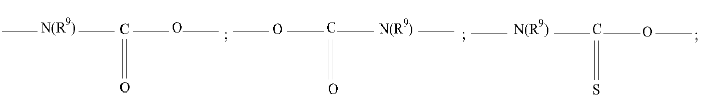 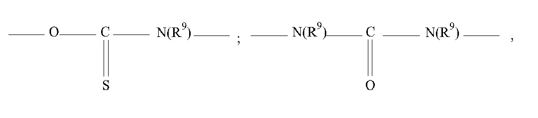 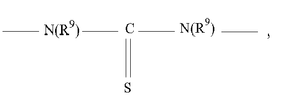 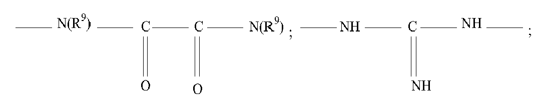 et 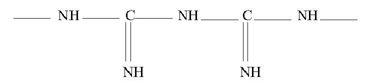 où R⁹ représente un atome d'hydrogène ou un groupe alkyle, linéaire ou ramifié, en C₁ à C₂₀, à condition qu'au moins 50 % des R⁹ du polymère représente un atome d'hydrogène et qu'au moins deux des groupes G du polymère soient un autre groupe que : 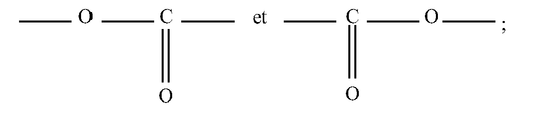
   6) n est un nombre entier allant de 2 à 500, de préférence de 2 à 200, et m est un nombre entier allant de 1 à 1000, de préférence de 1 à 700 et mieux encore de 6 à 200.
      Selon l'invention, 80 % des R⁴, R⁵, R⁶ et R⁷, du polymère sont choisis de préférence parmi les groupes méthyle, éthyle, phényle et 3,3,3-trifluoropropyle.
      Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :
      a) les groupes alkylène linéaires en C₁ à C₂₀, de préférence en C₁ à C₁₀,
      b) les groupes alkylène ramifiés pouvant comporter des cycles et des insaturations non conjuguées, en C₃₀ à C₅₆,
      c) les groupes cycloalkylène en C₅-C₆,
      d) les groupes phénylène éventuellement substitués par un ou plusieurs groupes alkyle en C₁ à C₄₀,
      e) les groupes alkylène en C₁ à C₂₀, comportant de 1 à 5 groupes amides,
      f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆,
      g) les chaînes polyorganosiloxane de formule : 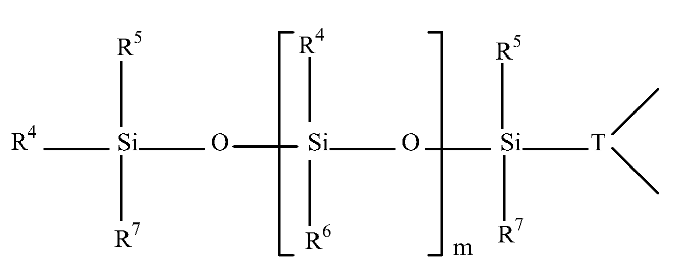 ou 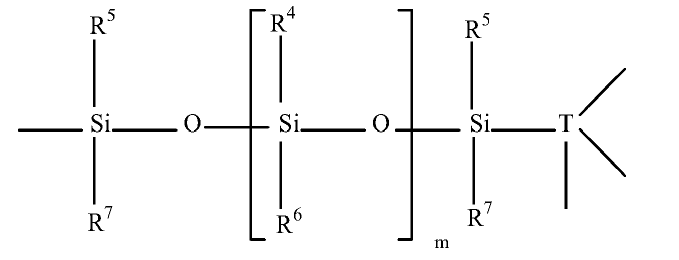
      dans laquelle R⁴, R⁵, R⁶, R⁷, T et m sont tels que définis ci-dessus.
B) Selon la seconde variante, les polyorganosiloxanes peuvent être des polymères comprenant au moins un motif répondant à la formule (II) : 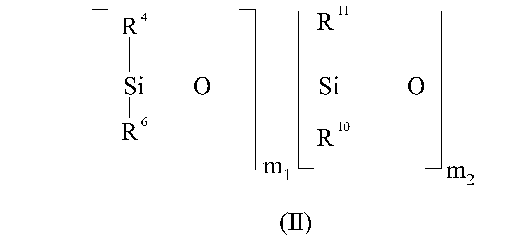
dans laquelle
- R⁴ et R⁶, identiques ou différents, sont tels que définis ci-dessus pour la formule (I),
- R¹⁰ représente un groupe tel que défini ci-dessus pour R⁴ et R⁶, ou représente le groupe de formule -X-G-R¹² dans laquelle X et G sont tels que définis ci-dessus pour la formule (I) et R¹² représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
- R¹¹ représente le groupe de formule -X-G-R¹² dans laquelle X, G et R¹² sont tels que définis ci-dessus,
- m₁ est un nombre entier allant de 1 à 998, et
- m₂ est un nombre entier allant de 2 à 500.

Selon l'invention, le polymère siliconé peut être un homopolymère, c'est-à-dire un polymère comportant plusieurs motifs identiques, en particulier des motifs de formule (I) ou de formule (II).

Selon l'invention, on peut aussi utiliser un polymère constitué par un copolymère comportant plusieurs motifs de formule (I) différents, c'est-à-dire un polymère dans lequel l'un au moins des R⁴, R⁵, R⁶, R⁷, X, G, Y, m et n est différent dans l'un des motifs. Le copolymère peut être aussi formé de plusieurs motifs de formule (II), dans lequel l'un au moins des R⁴, R⁶, R¹⁰, R¹¹, m₁ et m₂ est différent dans l'un au moins des motifs.

On peut encore utiliser un polymère comportant au moins un motif de formule (I) et au moins un motif de formule (II), les motifs de formule (I) et les motifs de formule (II) pouvant être identiques ou différents les uns des autres.

Selon une variante de l'invention, on peut encore utiliser un polymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des polymères blocs, des polymères séquencés ou des polymères greffés.

Selon un mode de réalisation avantageux de l'invention, les groupes capables d'établir des interactions hydrogènes sont des groupes amides de formule -C(O)NH- et - HN-C(O)-.

Dans ce cas, le polymère siliconé peut être un polymère comprenant au moins un motif de formule (III) ou (IV) : 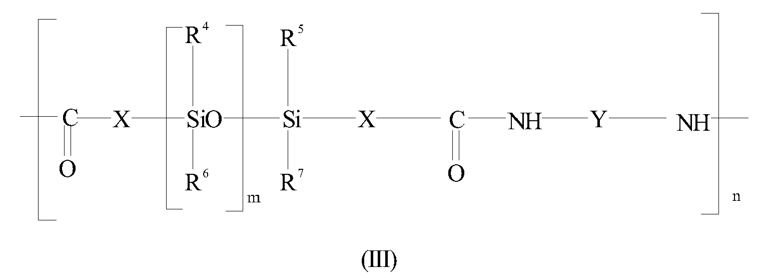 ou 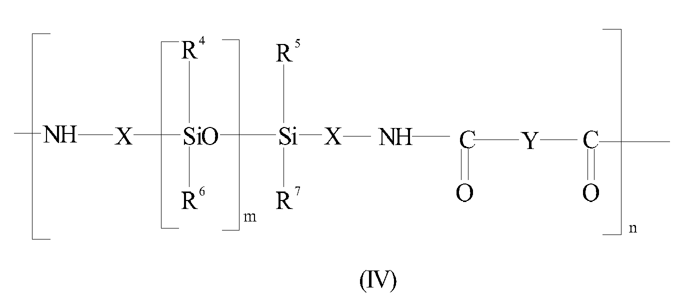 dans lesquelles R⁴, R⁵, R⁶, R⁷, X, Y, m et n sont tels que définis ci-dessus.

Un tel motif peut être obtenu :
- soit par une réaction de condensation entre un silicone à extrémités α, ω-acides carboxyliques et une ou plusieurs diamines, selon le schéma réactionnel suivant : 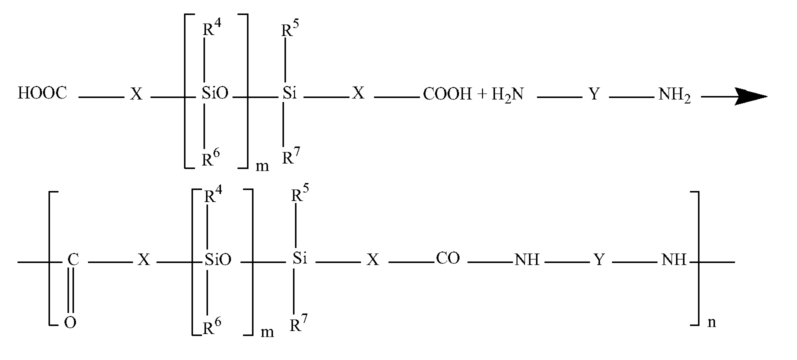
- soit par réaction de deux molécules d'acide carboxylique α-insaturé avec une diamine selon le schéma réactionnel suivant :

   CH₂=CH-X¹-COOH+H₂N-Y-NH₂ →CH₂=CH-X¹-CO-NH-Y-NH-CO-X¹-CH=CH₂
suivie de l'addition d'un siloxane sur les insaturations éthyléniques, selon le schéma suivant :

CH₂=CH-X¹-CO-NH-Y-NH-CO-X¹-CH=CH₂

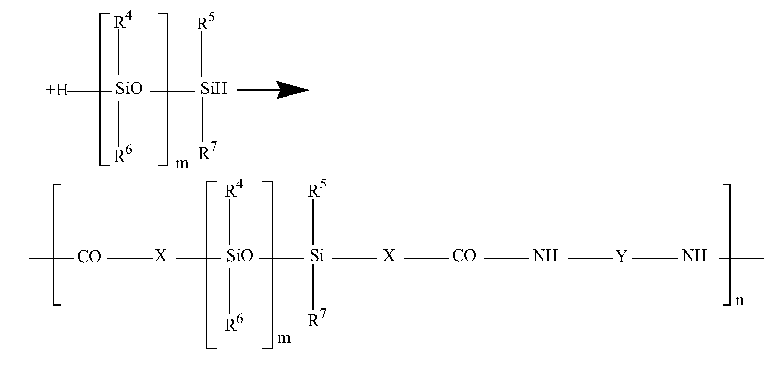

dans lesquels X¹-(CH₂)₂- correspond au X défini ci-dessus et Y, R⁴, R⁵, R⁶, R⁷ et m sont tels que définis ci-dessus,
- soit par réaction d'un silicone à extrémités α, ω-NH₂ et d'un diacide de formule HOOC-Y-COOH selon le schéma réactionnel suivant :

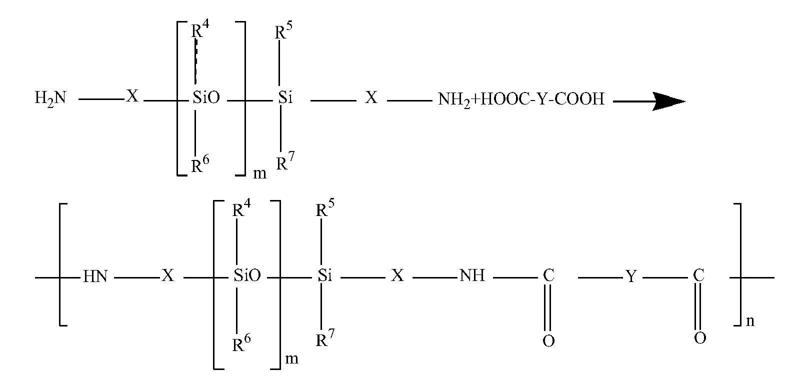

Dans ces polyamides de formule (III) ou (IV), m va de 1 à 700, en particulier de 15 à 500 et notamment de 50 à 200 et n va particulier de 1 à 500, de préférence de 1 à 100 et mieux encore de 4 à 25,
- X est de préférence une chaîne alkylène linéaire ou ramifiée ayant de 1 à 30 atomes de carbone, en particulier 1 à 20 atomes de carbone, notamment de 5 à 15 atomes de carbone et plus particulièrement de 10 atomes de carbone, et
- Y est de préférence une chaîne alkylène linéaire ou ramifiée ou pouvant comporter des cycles et/ou des insaturations, ayant de 1 à 40 atomes de carbone, en particulier de 1 à 20 atomes de carbone, et mieux encore de 2 à 6 atomes de carbone, en particulier de 6 atomes de carbone.

Dans les formules (III) et (IV), le groupe alkylène représentant X ou Y peut éventuellement contenir dans sa partie alkylène au moins l'un des éléments suivants :
1) 1 à 5 groupes amides, urée, uréthane, ou carbamate,
2) un groupe cycloalkyle en C₅ ou C₆, et
3) un groupe phénylène éventuellement substitué par 1 à 3 groupes alkyles identiques ou différents en C₁ à C₃.

Dans les formules (III) et (IV), les groupes alkylènes peuvent aussi être substitués par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

Dans ces formules (III) et (IV), Y peut aussi représenter : 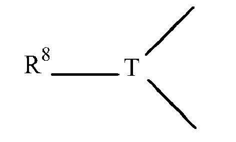 où R⁸ représente une chaîne polyorganosiloxane, et T représente un groupe de formule : 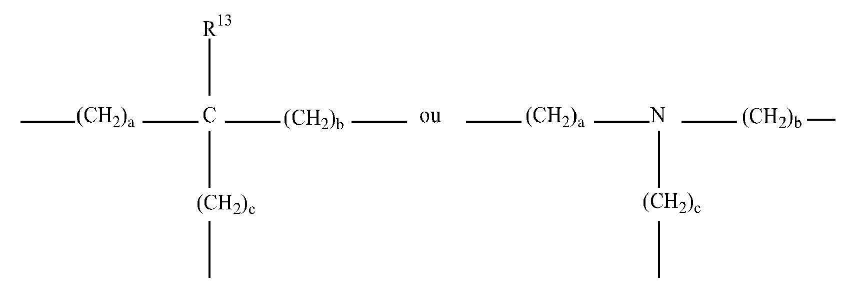 dans lesquelles a, b et c sont indépendamment des nombres entiers allant de 1 à 10, et R¹³ est un atome d'hydrogène ou un groupe tel que ceux définis pour R⁴, R⁵, R⁶ et R⁷.

Dans les formules (III) et (IV), R⁴, R⁵, R⁶ et R⁷ représentent de préférence, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (III) ou (IV) identiques ou différents.

Ainsi, le polymère peut être un polyamide contenant plusieurs motifs de formule (III) ou (IV) de longueurs différentes, soit un polyamide répondant à la formule (V) : 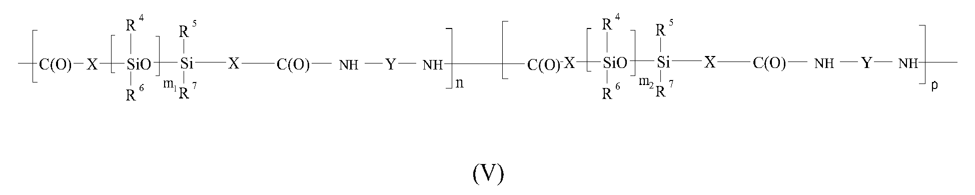 dans laquelle X, Y, n, R⁴ à R⁷ ont les significations données ci-dessus, m₁ et m₂ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le polymère peut répondre à la formule VI: 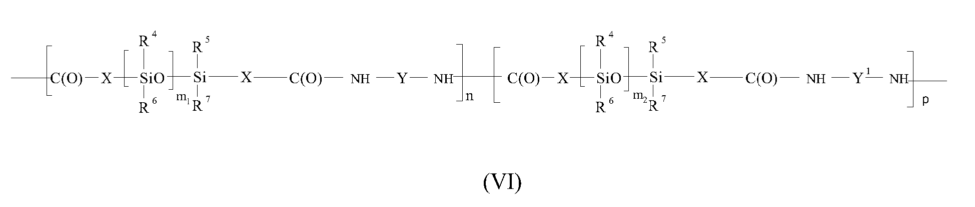 dans laquelle R⁴ à R⁷, X, Y, m₁, m₂, n et p ont les significations données ci-dessus et Y' est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, le polymère siliconé peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le polymère peut comprendre au moins un motif de formule (VII) : 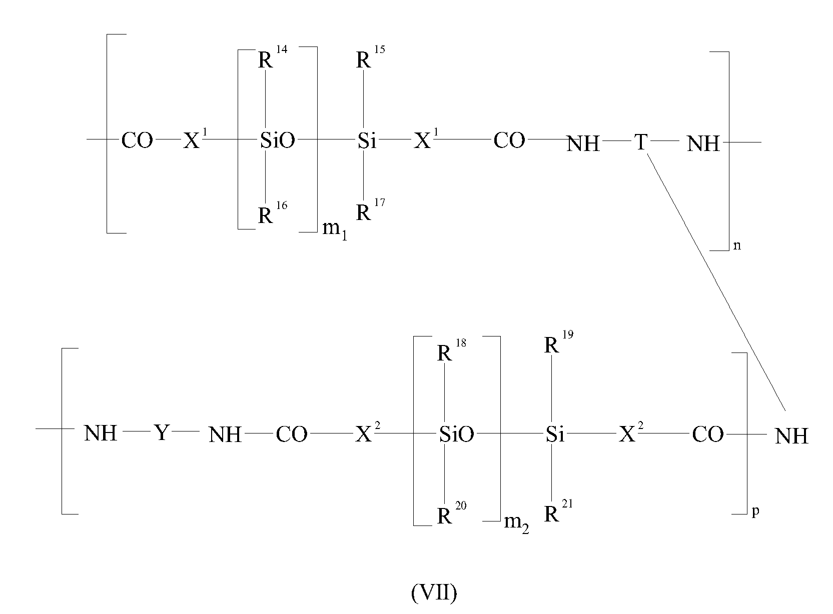 dans laquelle X¹ et X² qui sont identiques ou différents, ont la signification donnée pour X dans la formule (I), n est tel que défini dans la formule (I), Y et T sont tels que définis dans la formule (I), R¹⁴ à R²¹ sont des groupes choisis dans le même groupe que les R⁴ à R⁷, m₁ et m₂ sont des nombres situés dans la gamme allant de 1 à 1 000, et p est un nombre entier allant de 2 à 500.

Dans la formule (VII), on préfère que :
- p varie de 1 à 25, mieux encore de 1 à 7,
- R¹⁴ à R²¹ soient des groupes méthyle,
- T réponde à l'une des formules suivantes : 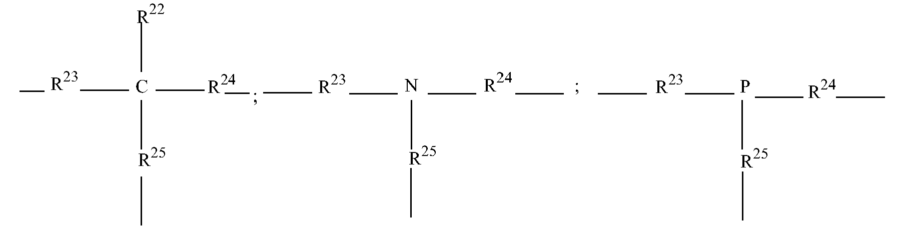 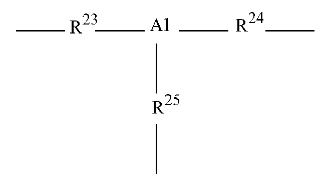
dans lesquelles R²² est un atome d'hydrogène ou un groupe choisi parmi les groupes définis ci-dessus pour R⁴ à R⁷, et R²³, R²⁴ et R²⁵ sont indépendamment des groupes alkylène, linéaires ou ramifiés, de préférence encore, à la formule : 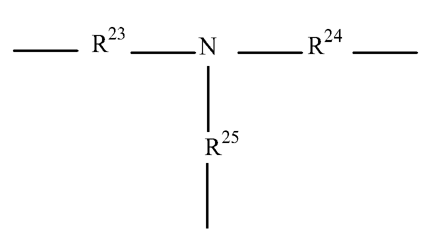 en particulier avec R²³, R²⁴ et R²⁵ représentant -CH₂-CH₂-,
- m₁ et m₂ vont de 15 à 500, et mieux encore de 15 à 45,
- X₁ et X₂ représentent -(CH₂)₁₀-, et
- Y représente -CH₂-.

Ces polyamides à motif silicone greffé de formule (VII) peuvent être copolymérisés avec des polyamides-silicones de formule (II) pour former des copolymères blocs, des copolymères alternés ou des copolymères aléatoires. Le pourcentage en poids de motifs silicone greffé (VII) dans le copolymère peut aller de 0,5 à 30 % en poids.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon une variante de réalisation de l'invention, on peut utiliser un copolymère de polyamide silicone et de polyamide hydrocarboné, soit un copolymère comportant des motifs de formule (III) ou (IV) et des motifs polyamide hydrocarboné. Dans ce cas, les motifs polyamide-silicone peuvent être disposés aux extrémités du polyamide hydrocarboné.

Avantageusement, la composition selon l'invention comprend au moins un polymère bloc polydiméthylsiloxane de formule générale (I) possédant un indice m de valeur supérieure à 50, en particulier supérieure à 75, mieux supérieure à 100 et notamment d'environ 120.

Selon un mode de réalisation particulier, la composition comprend au moins un polymère bloc polydiméthylsiloxane de formule générale (I) possédant un indice m de valeur environ 15.

En particulier, la composition selon l'invention comprend :
- au moins un premier polymère comprenant au moins un motif de formule générale (I) où m varie de 50 à 600, en particulier de 60 à 400, notamment de 75 à 200 et plus particulièrement est de l'ordre de 120 et
- au moins un second polymère comprenant au moins un motif de formule générale (I) où m va de 5 à 100, en particulier de 10 à 75 et plus particulièrement est de l'ordre de 15 .

De préférence encore, la composition selon l'invention comprend :
- au moins un premier polymère comprenant au moins un motif de formule (III) où m varie de 50 à 600, en particulier de 60 à 400, notamment de 75 à 200 et plus particulièrement est de l'ordre de 120 et
- au moins un second polymère comprenant au moins un motif de formule (III) où m va de 5 à 100, en particulier de 10 à 75 et plus particulièrement est de l'ordre de 15.

Ainsi, l'invention a également pour objet une composition cosmétique pour les lèvres comprenant dans un milieu physiologiquement acceptable au moins un premier polymère siliconé et au moins un second polymère siliconé comprenant chacun indépendamment au moins un motif de formule (III) telle que définie plus haut dans laquelle :
- m varie de 50 à 600, en particulier de 60 à 400, notamment de 75 à 200 et plus particulièrement est de l'ordre de 120 pour le premier polymère et
- m va de 5 à 100, en particulier de 10 à 75 et plus particulièrement est de l'ordre de 15 pour le second polymère.

De préférence, ces premier et second polymères répondent à la formule (III) dans laquelle R⁴, R⁵, R⁶ et R⁷ représentent indépendamment, un groupe alkyle en C₁ à C₄₀, linéaire ou ramifié, de préférence un groupe CH₃, C₂H₅, n-C₃H₇ ou isopropyle.

De préférence encore, X et Y représentent indépendamment un groupe choisi parmi les groupes alkylène linéaires en C₁ à C₂₀, de préférence en C₁ à C₁₀.

De préférence le premier polymère a une masse moléculaire moyenne en poids comprise entre de 1 000 et 500 000 g/mol, notamment entre 10 000 et 300 000 g/mol et le second polymère a de préférence une masse moléculaire moyenne en poids allant de 50 000 à 500 000 g/mol.

Avantageusement, le rapport entre le premier polymère et le deuxième polymère va de 5/95 à 95/5, de préférence de 70/30 à 30/70.

A titre d'exemples de premier polymère utilisable, on peut citer un des polyamides siliconés, obtenus conformément aux exemples 1 à 3 du document US-A-5 981 680.

Selon une variante de réalisation de l'invention, le polymère est constitué par un homopolymère ou copolymère comportant des groupes uréthane ou urée. Ces polymères sont décrits en détail dans la demande WO 2003/106614.

Comme précédemment, un tel polymère peut comporter des motifs polyorganosiloxanes contenant deux ou plusieurs groupes uréthanes et/ou urées, soit dans le squelette du polymère, soit sur des chaînes latérales ou comme groupes pendants.

Les polymères comportant au moins deux groupes uréthanes et/ou urées dans le squelette peuvent être des polymères comprenant au moins un motif répondant à la formule suivante (VIII) : 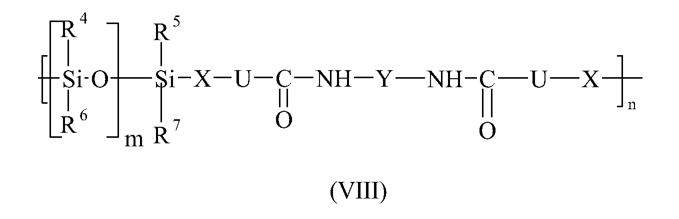 dans laquelle les R⁴, R⁵, R⁶, R⁷, X, Y, m et n ont les significations données ci-dessus pour la formule (I), et U représente -O- ou -NH-, afin que : 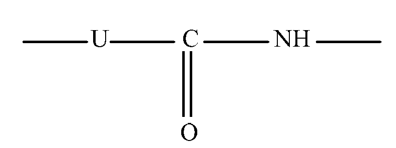 corresponde à un groupe uréthane ou urée.

Dans cette formule (VIII), Y peut être un groupe alkylène, en C₁ à C₄₀, linéaire ou ramifié, substitué éventuellement par un groupe alkyle en C₁ à C₁₅ ou un groupe aryle en C₅ à C₁₀. De préférence, on utilise un groupe -(CH₂)₆-.

Y peut aussi représenter un groupe cycloaliphatique ou aromatique en C₅ à C₁₂ pouvant être substitué par un groupe alkyle en C₁ à C₁₅ ou un groupe aryle en C₅ à C₁₀, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane. Généralement, on préfère que Y représente un radical alkylène en C₁ à C₄₀, linéaire ou ramifié, ou un radical cycloalkylène en C₄ à C₁₂.

Y peut aussi représenter une séquence polyuréthane ou polyurée correspondant à la condensation de plusieurs molécules de diisocyanate avec une ou plusieurs molécules de coupleurs du type diol ou diamine. Dans ce cas, Y comprend plusieurs groupes uréthane ou urée dans la chaîne alkylène.
Il peut répondre à la formule (IX) : 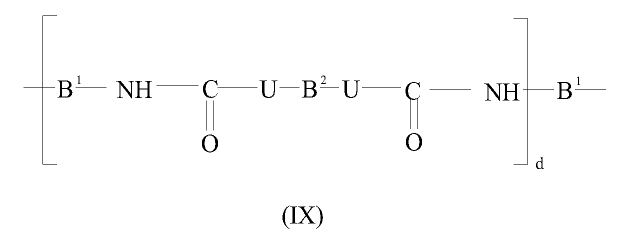 dans laquelle B¹ est un groupe choisi parmi les groupes donnés ci-dessus pour Y, U est -O- ou -NH-, et B² est choisi parmi :
- les groupes alkylène en C₁ à C₄₀, linéaires ou ramifiés,
- les groupes cycloalkylène en C₅ à C₁₂, éventuellement porteurs de substituants alkyle, par exemple un à trois groupes méthyle ou éthyle, ou alkylène, par exemple le radical du diol : cyclohexane diméthanol,
- les groupes phénylène pouvant éventuellement être porteurs de substituants alkyles en C₁ à C₃, et
- les groupes de formule : 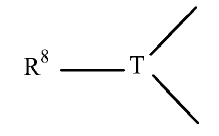
dans laquelle T est un radical trivalent hydrocarboné pouvant contenir un ou plusieurs hétéroatomes tels que l'oxygène, le soufre et l'azote et R⁸ est une chaîne polyorganosiloxane ou une chaîne alkyle en C₁ à C₅₀, linéaire ou ramifiée.

T peut représenter par exemple : 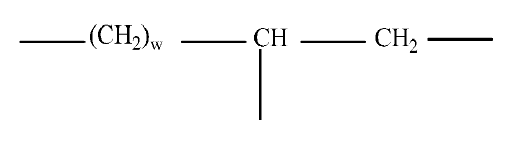 ou 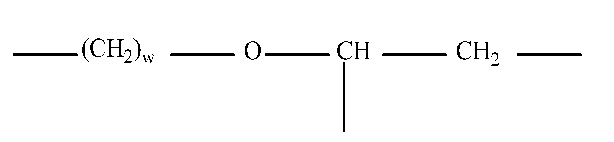 avec w étant un nombre entier allant de 1 à 10 et R⁸ étant une chaîne polyorganosiloxane.

Lorsque Y est un groupe alkylène, en C₁ en C₄₀ linéaire ou ramifié, on préfère les groupes -(CH₂)₂- et -(CH₂)₆-.

Dans la formule donnée ci-dessus pour Y, d peut être un entier allant de 0 à 5, de préférence de 0 à 3, de préférence encore égal à 1 ou 2.

De préférence B² est un groupe alkylène en C₁ à C₄₀, linéaire ou ramifié, en particulier -(CH₂)₂- ou -(CH₂)₆-, ou le groupe : 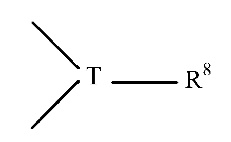 avec R⁸ étant une chaîne polyorganosiloxane.

Comme précédemment, le polymère constituant le polymère siliconé peut être formé de motifs silicone uréthane et/ou silicone-urée de longueur et/ou de constitution différentes, et se présenter sous la forme de copolymères blocs, séquencés ou statistiques (aléatoires).

Les polymères de formule (VIII) comportant des groupes urées ou uréthanes dans la chaîne du polymère siliconé peuvent être obtenus par réaction entre un silicone à groupes terminaux α,ω-NH₂ ou -OH, de formule : 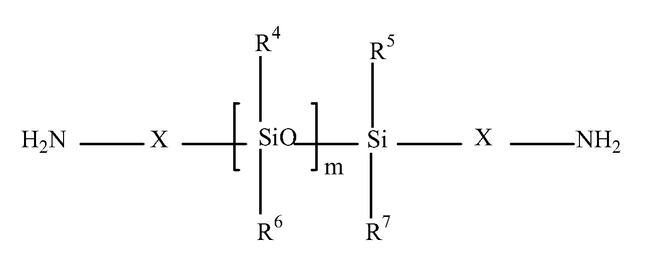 dans laquelle m, R⁴, R⁵, R⁶, R⁷ et X sont tels que définis pour la formule (I), et un diisocyanate OCN-Y-NCO où Y a la signification donnée dans la formule (I) ; et éventuellement un coupleur diol ou diamine de formule H₂N-B²-NH₂ ou HO-B² -OH, où B² est tel que défini dans la formule (IX).

Suivant les proportions stoechiométriques entre les deux réactifs, diisocyanate et coupleur, on pourra avoir pour Y la formule (IX) avec d égale 0 où d égale 1 à 5.

Comme dans le cas des polyamides silicones de formule (IV), (II) ou (III), on peut utiliser dans l'invention des polyuréthanes ou des polyurées silicones ayant des motifs de longueur et de structure différentes, en particulier des motifs de longueurs différentes par le nombre d'unités silicones. Dans ce cas, le copolymère peut répondre par exemple à la formule : 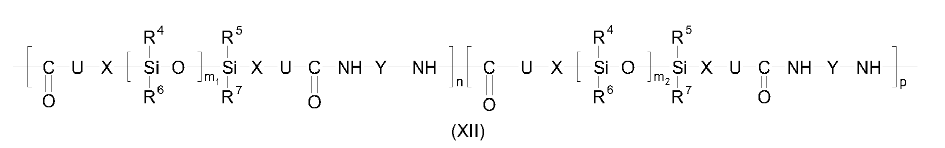 dans laquelle R⁴, R⁵, R⁶, R⁷, X, Y et U sont tels que définis pour la formule (VIII) et m₁, m₂, n et p sont tels que définis pour la formule (V).

Selon l'invention, le silicone peut aussi comporter les groupes uréthane et/ou urée non plus dans le squelette mais en ramifications latérales.

Dans ce cas, le polymère peut comprendre au moins un motif de formule : 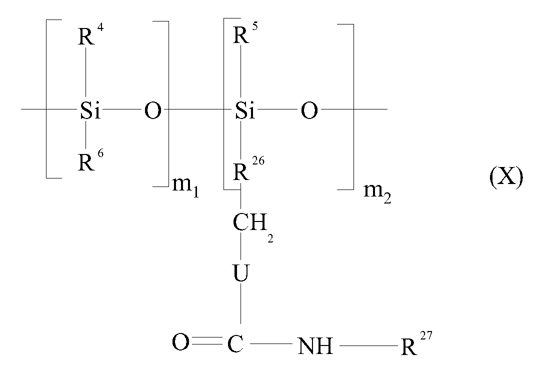 dans laquelle R⁴, R⁶, R⁵, m₁ et m₂ ont les significations données ci-dessus pour la formule (II), et R⁵ pour la formule (I),
- U représente O ou NH,
- R²⁶ représente un groupe alkylène en C₁ à C₄₀, comportant éventuellement un ou plusieurs hétéroatomes choisis parmi O et N, ou un groupe phénylène, et
- R²⁷ est choisi parmi les groupes alkyle en C₁ à C₅₀, linéaires, ramifiés ou cycliques, saturés ou insaturés, et les groupes phényle éventuellement substitués par un à trois groupes alkyles en C₁ à C₃.

Les polymères comportant au moins un motif de formule (X) contiennent des unités siloxanes et des groupes urées ou uréthanes, et ils peuvent être utilisés comme polymère siliconé dans les compositions de l'invention.

Les polymères siloxanes peuvent avoir un seul groupe urée ou uréthane par ramification ou peuvent avoir des ramifications à deux groupes urée ou uréthane, ou encore contenir un mélange de ramifications à un groupe urée ou uréthane et de ramifications à deux groupes urée ou uréthane.

Ils peuvent être obtenus à partir de polysiloxanes ramifiés, comportant un ou deux groupes amino par ramification, en faisant réagir ces polysiloxanes avec des monoisocyanates.

A titre d'exemples de polymères de départ de ce type ayant des ramifications amino et diamino, on peut citer les polymères répondant aux formules suivantes :

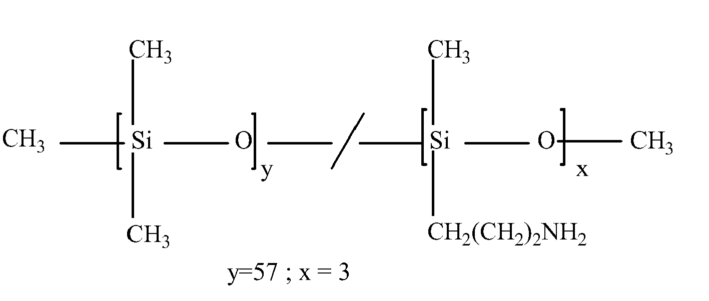

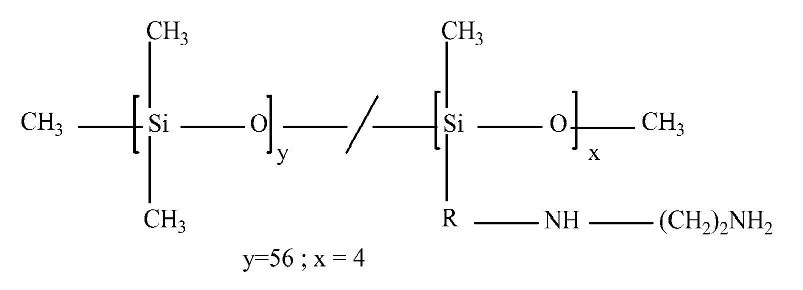

Dans ces formules, le symbole "/" indique que les segments peuvent être de longueurs différentes et dans un ordre aléatoire, et R représente un groupe aliphatique linéaire ayant de préférence 1 à 6 atomes de carbone et mieux encore 1 à 3 atomes de carbone.

De tels polymères à ramification peuvent être formés en faisant réagir un polymère siloxane, ayant au moins trois groupes amino par molécule de polymère, avec un composé ayant un seul groupe monofonctionnel (par exemple un acide, un isocyanate ou isothiocyanate) pour faire réagir ce groupe monofonctionnel avec l'un des groupes amino et former les groupes capables d'établir des interactions hydrogène. Les groupes amino peuvent être sur des chaînes latérales s'étendant de la chaîne principale du polymère siloxane de sorte que les groupes capables d'établir des interactions hydrogène sont formés sur ces chaînes latérales, ou bien les groupes amino peuvent être aux extrémités de la chaîne principale de sorte que les groupes capables d'interaction hydrogène seront des groupes terminaux du polymère.

Comme mode opératoire pour former un polymère contenant des unités siloxanes et des groupes capables d'établir des interactions hydrogène, on peut citer la réaction d'une siloxane diamine et d'un diisocyanate dans un solvant siliconé de façon à fournir directement un gel. La réaction peut être exécutée dans un fluide siliconé, le produit résultant étant dissous dans le fluide siliconé, à température élevée, la température du système étant ensuite diminuée pour former le gel.

Les polymères préférés pour l'incorporation dans les compositions selon la présente invention, sont des copolymères siloxanes-urées qui sont linéaires et qui contiennent des groupes urées comme groupes capables d'établir des interactions hydrogène dans le squelette du polymère.

A titre d'illustration d'un polysiloxane terminé par quatre groupes urées, on peut citer le polymère de formule : 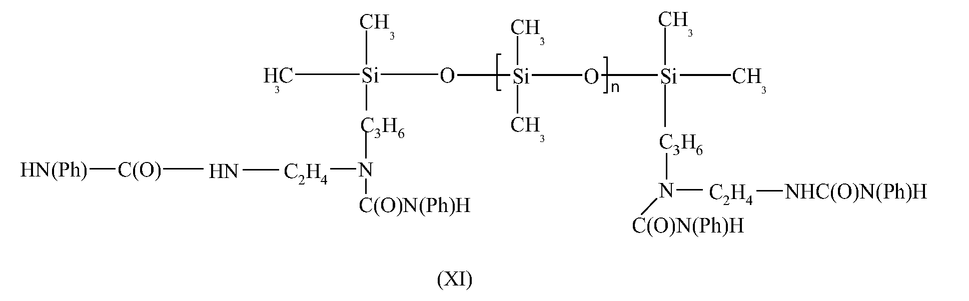 où Ph est un groupe phényle et n est un nombre de 0 à 300, en particulier de 0 à 100, par exemple de 50.

Ce polymère est obtenu par réaction du polysiloxane à groupes amino suivant : 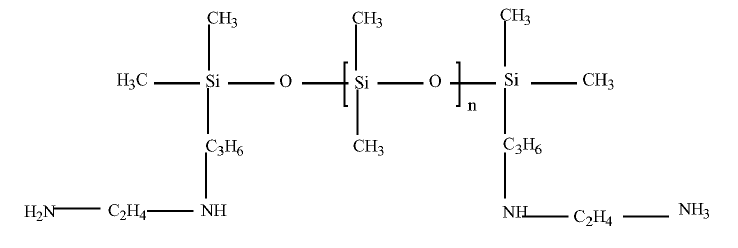 dans lequel n est défini tel que pour la formule (XI),
avec l'isocyanate de phényle.

On peut obtenir également des polyuréthanes ou polyurées silicones ramifiés en utilisant à la place du diisocyanate OCN-Y-NCO, un triisocyanate de formule :

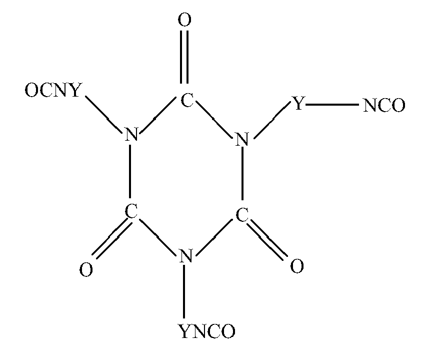

On obtient ainsi une polyuréthane ou polyurée silicone ayant des ramifications comportant une chaîne organosiloxane avec des groupes capables d'établir des interactions hydrogène. Un tel polymère comprend par exemple un motif répondant à la formule : 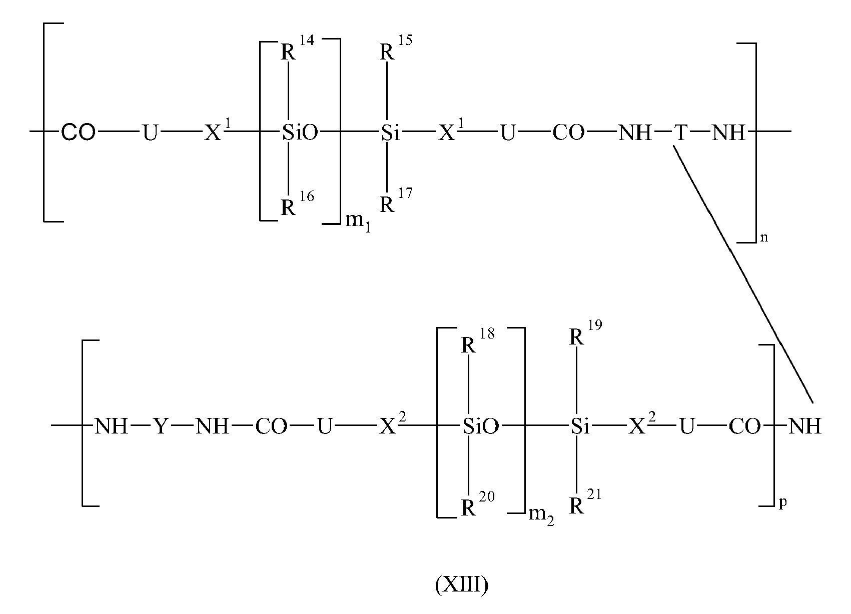 dans laquelle X¹ et X² qui sont identiques ou différents, ont la signification donnée pour X dans la formule (I), n est tel que défini dans la formule (I), Y et T sont tels que définis dans la formule (I), R¹⁴ à R²¹ sont des groupes choisis dans le même groupe que les R⁴ à R⁷, m₁ et m₂ sont des nombres situés dans la gamme allant de 1 à 1 000, et p est un nombre entier allant de 2 à 500.

Comme dans le cas des polyamides, ce copolymère peut comporter aussi des motifs polyuréthanes silicones sans ramification.

Les polyurées et les polyuréthanes à base de siloxanes préférés sont :
- les polymères de formule (VIII) où m varie de 50 à 600, en particulier de 60 à 400, notamment de 75 à 200 et plus particulièrement de l'ordre de 100 ;
- des mélanges de polymères de formule (VIII) combinant :
   1) 80 à 99 % en poids d'un polymère de formule (VIII) où m varie de 50 à 600, en particulier de 60 à 400, notamment de 75 à 200 et plus particulièrement de l'ordre de 100, et
   2) 1 à 20 % d'un polymère de formule (VIII) où m est dans la gamme de 5 à 100, en particulier de 10 à 75 et plus particulièrement est de l'ordre de 15;
- des mélanges de polymères de formule (VIII) combinant :
   1) 80 à 99 % en poids de polymères de formule (VIII) où n est égal à 2 à 10, en particulier 3 à 6, et
   2) 1 à 20 % de polymères de formule (VIII) où n est dans la gamme de 5 à 500, en particulier de 30 à 100 ;
- des mélanges de polymères de formule (VIII) combinant :
   1) 1 à 20 % en poids de polymères de formule (VIII), où n est égal à 2 à 10, en particulier 3 à 6, et
   2) 80 à 99 % de polymères de formule (VIII), où n est dans la gamme de 30 à 500, en particulier de 30 à 100 ;
- des polymères de formule (VIII) où X représente un radical alkyle en C₃ à C₁₅ en particulier en C₅ à C₁₂ et notamment en C₁₀ et
- des polymères de formule (VIII) où Y représente un radical alkyle en C₃ à C₁₀ en particulier en C₄ à C₈ et notamment en C₆.

Comme dans le cas des polyamides, on peut utiliser dans l'invention des copolymères de polyuréthane -ou de polyurée- silicone et de polyuréthane ou polyurée hydrocarboné en réalisant la réaction de synthèse du polymère en présence d'une séquence α, ω-difonctionnelle de nature non silicone, par exemple un polyester, un polyéther ou une polyoléfine.

Comme on l'a vu précédemment, les polymères siliconés de l'invention peuvent avoir des motifs siloxanes dans la chaîne principale du polymère et des groupes capables d'établir des interactions hydrogène, soit dans la chaîne principale du polymère ou aux extrémités de celle-ci, soit sur des chaînes latérales ou ramifications de la chaîne principale. Ceci peut correspondre aux cinq dispositions suivantes : 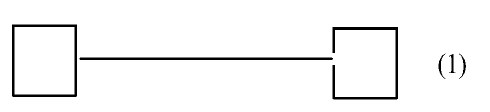 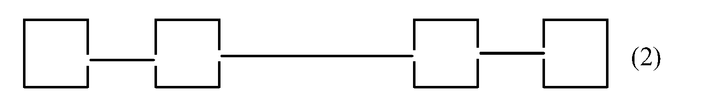 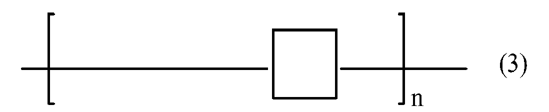 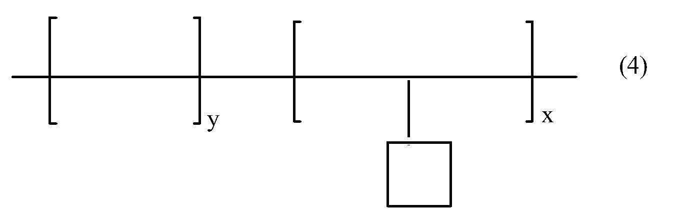 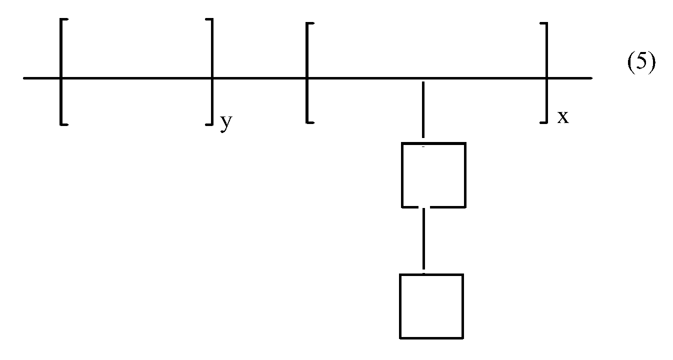 dans lesquelles, la ligne continue est la chaîne principale du polymère siloxane et les carrés représentent les groupes capables d'établir des interactions hydrogène.

Dans le cas (1), les groupes capables d'établir des interactions hydrogène sont disposés aux extrémités de la chaîne principale. Dans le cas (2), deux groupes capables d'établir des interactions hydrogène, sont disposés à chacune des extrémités de la chaîne principale.

Dans le cas (3), les groupes capables d'établir des interactions hydrogène sont disposés à l'intérieur de la chaîne principale dans des motifs répétitifs.

Dans les cas (4) et (5), il s'agit de copolymères dans lesquels les groupes capables d'établir des interactions hydrogène sont disposés sur des ramifications de la chaîne principale d'une première série de motifs qui sont copolymérisés avec des motifs ne comportant pas de groupes capables d'établir des interactions hydrogène.

Les polymères et copolymères utilisés dans la composition de l'invention ont avantageusement une température de transition de l'état solide à l'état liquide allant de 45 °C à 190 °C. De préférence, ils présentent une température de transition de l'état solide à l'état liquide allant de 70 à 130 °C et mieux de 80 °C à 105 °C.

Le ou les polymères siliconés peuvent être présents dans la composition selon l'invention en une teneur totale allant de 0,5 % à 70 % en poids par rapport au poids total de la composition, de préférence allant de 5 % à 50 % en poids, et mieux allant de 10 % à 45 % en poids.

### Tensioactif phosphaté

La composition selon l'invention comprend au moins un tensioactif phosphaté.

La présence du tensioactif phosphaté permet avantageusement de conférer aux compositions de l'invention un pouvoir glissant amélioré à l'application et une meilleure homogénéité. Ainsi, les compositions de l'invention sont plus agréables à porter.

Dans le cadre de l'invention, un tensioactif phosphaté convenant à la mise en oeuvre des compositions de la présente invention est avantageusement dénué de fonction organogélatrice.

Un tensioactif phosphaté convenant à l'invention est également avantageusement dépourvu de fonction plastifiante.

Par tensioactif phosphaté, on entend un tensioactif dont la partie polaire comprend au moins un groupement phosphate. Le tensioactif phosphaté peut être choisi parmi les composés suivants et leurs mélanges.

Le tensioactif phosphaté peut notamment être de formule (XIV) : 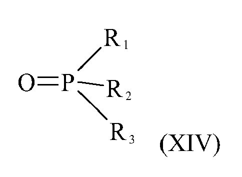 avec R₁, R₂ et R₃ identiques ou différents et pouvant être choisis parmi :
- un groupement OM avec M représentant un métal alcalin, tel que Na, Li, K, de préférence Na ou K,
- un groupe OR₄, dans lequel R₄ représente un groupe alkyle en C₅-C₄₀ linéaire, ramifié, cyclique ou aromatique,
- un groupement OH, et
- un groupe oxyéthyléné (OCH₂CH₂)ₙ(OCH₂CHCH₃)ₘOR avec R représentant un atome hydrogène ou un groupe alkyle, linéaire ou ramifié, en C₁ à C₂₀, en particulier en C₅ à C₁₈, voire en C₁₂ à C₁₅, et n et m étant des nombres entiers avec n variant de 1 à 50, voire étant égal à 10, et m variant de 0 à 50 voire étant égal à 0.

Selon un premier mode de réalisation, R₁, R₂ et R₃ peuvent être identiques.

Selon un autre mode de réalisation, au moins un, notamment deux, voire l'ensemble des radicaux R₁, R₂ et R₃ figure(nt), un groupement OR₄, dans lequel R₄ représente un groupe alkyle, linéaire ou ramifié, en C₁₀ à C₃₀, en particulier en C₁₅ à C₂₁, voire en C₁₈, R₁, R₂ et R₃ peuvent être identiques ou différents.

A titre illustratif et non limitatif de ces composés de formule (XIV), on peut notamment citer le phosphate de trioleyle tel que le NIKKOL TDP commercialisé par la société NIKKO CHEMICALS, le mélange de triesters d'acide phosphorique et d'ether d'ethylène glycol et d'alcools gras en C₁₂-C₁₅ (environ 10 OE) (nom INCI TRI-C₁₂-₁₅ PARETH-10 PHOSPHATE) tel que le NIKKOL TDP-10 commercialisé par la société NIKKO CHEMICALS et le cetyl phosphate de potassium tel que l'AMPHISOL K commercialisé par la société DSM Nutricionnal Products ou l'ARLATONE MAP 160 K de Uniquema.

La composition peut également comprendre à titre de tensioactif phosphaté au moins un glycérophospholipide, le cas échéant en mélange avec un composé de formule (XIV).

Au sens de la présente invention, on entend désigner par glycérophospholipide, un ester obtenu par réaction du glycérol avec au moins un acide gras saturé ou insaturé et l'acide phosphorique, ledit acide phosphorique étant substitué par un composé choisi parmi les alcools portant une fonction amine, notamment un bêta-amino alcool. Le bêta-amino alcool peut être choisi, par exemple, parmi la choline, l'éthanolamine et/ou la sérine.

Le glycérophospholipide peut notamment être défini selon la formule générale (XV) suivante : 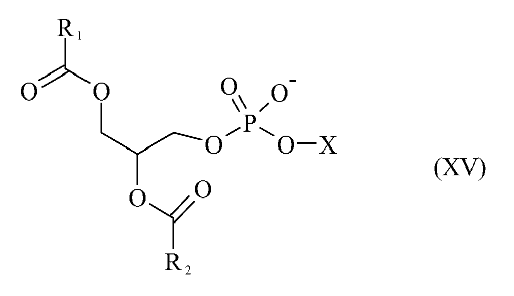 dans laquelle :
- R₁ et R₂ représentent, indépendamment l'un de l'autre, un acide gras comprenant de 4 à 24 atomes de carbone, saturé ou insaturé, le cas échéant ramifié, et susceptible d'être substitué par une ou plusieurs fonctions hydroxy et/ou amines, et
- X représente un substituant de formule générale R₃R₄R₅N⁺-CH(R₆)-CH₂- dans lequel R₃, R₄, R₅ et R₆ représentent, indépendamment l'un de l'autre, un hydrogène, des groupes alkyles comprenant de 1 à 6 atomes de carbone, et/ou une fonction carboxy. Notamment, X peut être choisi parmi la choline, la sérine ou l'éthanolamine.

Selon une variante de réalisation, R₁ et R₂, indépendamment l'un de l'autre, sont avantageusement choisis parmi l'acide butyrique, l'acide caproïque, l'acide caprylique, l'acide caprique, l'acide caproléïque, l'acide laurique, l'acide lauroléïque, l'acide myristique, l'acide myristoléique, l'acide palmitique, l'acide palmitoléique, l'acide stéarique, l'acide oléique, l'acide linoléique, l'acide linolénique, l'acide arachidique, l'acide isostéarique, l'acide dihydroxystéarique et l'acide ricinoléique.

Le glycérophospholipide peut être également un mélange de composés de formule générale (XV).

Le glycérophospholipide convenant à la mise en oeuvre de l'invention peut notamment comprendre de la phosphatidylcholine, de la phosphatidyléthanolamine et/ou de la phosphatidylsérine.

Selon un mode de réalisation, le glycérophospholipide comprend avantageusement un ester de glycérol, d'acide gras insaturé, d'acide phosphorique et de choline, également dénommé phosphatidylcholine (PC).

Le glycérophospholipide convenant à la mise en oeuvre de l'invention peut provenir d'une lécithine. Notamment, la lécithine peut comprendre, majoritairement, en tant que glycérophospholipide, de la phosphatidylcholine.

La phosphatidylcholine (PC) convenant à la mise en oeuvre des compositions conformes à l'invention peut être d'origine « naturelle » ou « synthétique ».

La PC dite « naturelle » peut être obtenue par extraction à partir de sources animales ou végétales, comme le soja, le tournesol, ou les oeufs. La phosphatidylcholine non hydrogénée obtenue naturellement, comme par exemple à partir de soja, contient généralement comme acide gras estérifiant le glycérol, de l'acide palmitique, de l'acide stéarique, de l'acide palmitoléique, de l'acide oléique, de l'acide linoléique, de l'acide linolénique, et éventuellement des acides gras en C₂₀ à C₂₂.

Par « phosphatidylcholine synthétique », on entend désigner au sens de la présente invention, de la phosphatidylcholine comportant au moins un acide gras distinct de ceux susceptibles d'être présents dans les PC dites naturelles.

Par « PC synthétique », on entend également désigner de la PC dite naturelle soumise à des modifications, telles que l'hydrogénation partielle, c'est-à-dire que seule une fraction des doubles liaisons présentes sur les acides gras insaturés est maintenue.

Parmi les sources de phosphatidylcholine plus ou moins purifiée convenant à la mise en oeuvre des compositions cosmétiques conformes à la présente invention, il peut être fait mention de EMULMETIK 930 commercialisée par la société LUCAS MEYER.

Le glycérophospholipide convenant à la mise en oeuvre de la présente invention peut être introduit dans la composition sous la forme d'une lécithine. Celle-ci est généralement obtenue par extraction lipidique au moyen de solvants apolaires, à partir de matières grasses végétales ou animales. Cette fraction lipidique comprend, habituellement, majoritairement, des glycérophospholipides, dont la phosphatidylcholine ou la phosphatidyléthanolamine.

Les lécithines convenant à la mise en oeuvre de la présente invention peuvent être des lécithines issues du soja, du tournesol, de l'oeuf et/ou leurs mélanges.

Les lécithines sont habituellement fournies sous forme dissoutes dans des acides gras, des triglycérides ou d'autres solvants, ou sous forme de poudres ou de pains.

Ce sont usuellement des mélanges de lécithines, dont la teneur en glycérophospholipides, dans les produits tels que commercialisés, varie de manière générale d'environ au moins 15 % à environ au moins 95 %.

De manière avantageuse, la lécithine utilisée comme matière première pour la préparation de la composition selon l'invention comprend au moins 45 % en poids, en particulier au moins 65 % en poids, en particulier au moins 75 % en poids, en particulier au moins 85 % en poids, et plus particulièrement au moins 95 % en poids de glycérophospholipide par rapport au poids total de la lécithine.

Parmi les lécithines pouvant convenir à la mise en oeuvre des compositions cosmétiques conformes à la présente invention, il peut être fait mention des lécithines commercialisées sous les références NATTERMANN PHOSPHOLIPID^{®}, PHOSPHOLIPON 80^{®} et PHOSALE 75^{®} par la société AMERICAN LECITHIN COMPANY, EPIKURON 145V, TOPCITHIN 300, EMULMETIK 930 et OVOTHIN 200 commercialisées par la société LUCAS MEYER.

Le glycérophospholipide peut en particulier être un glycérophospholipide non hydrogéné, c'est-à-dire un ester obtenu par réaction du glycérol avec au moins un acide gras insaturé et l'acide phosphorique, ledit acide phosphorique étant substitué par un composé choisi parmi les alcools portant une fonction amine, notamment un beta-amino alcool.

Par « insaturé » ou « insaturation », on entend désigner la présence d'au moins une, voire de plusieurs, doubles ou triples liaisons entre deux atomes de carbone.

Le glycérophospholipide peut en particulier être de phosphatidylcholine ou de la lécithine.

Le tensioactif phosphaté peut être choisi parmi le phosphate de trioléyle, le mélange de triesters d'acide phosphorique et d'éther d'ethylène glycol et d'alcools gras en C₁₂-C₁₅ (environ 10 OE), le cetyl phosphate de potassium, le cetyl phosphate, la lécithine et leurs mélanges.

Le tensioactif phosphaté est avantageusement un tensioactif non ionique tel que le phosphate de trioléyle, le mélange de triesters d'acide phosphorique et d'éther d'ethylène glycol et d'alcools gras en C₁₂-C₁₅ (environ 10 OE).

Le tensioactif phosphaté peut être présent dans la composition selon l'invention à une teneur comprise entre 0,1 et 30 % notamment entre 0,1 et 10 % voire entre 0,5 et 5 % en poids par rapport au poids total de la composition.

Selon une première variante, la composition selon l'invention peut associer au moins du phosphate de trioléyle et un polyamide/polydiméthylsiloxane.

Selon une deuxième variante, la composition selon l'invention associe au moins du phosphate de trioléyle et un polymère siliconé, comprenant au moins un motif de formule (III) telle que défini précédemment.

Selon une troisième variante, la composition selon l'invention associe au moins de la lécithine et un polyamide/polydiméthylsiloxane.

Selon une quatrième variante, la composition selon l'invention associe au moins de la lécithine et un polymère siliconé, comprenant au moins un motif de formule (III) telle que défini précédemment.

### Actifs

La composition selon l'invention peut comprendre en outre au moins un actif. Par « actif », on entend un composé ayant un effet cosmétique et/ou dermatologique sur les lèvres *via* un mécanisme physiologique.

Cet actif peut être hydrophile ou hydrophobe. L'actif peut être hydrosoluble.

Ainsi, l'actif présent dans la composition selon l'invention peut être indépendamment choisi parmi :
- les agents dermorelaxants,
- les agents stimulant la synthèse de macromolécules dermiques ou épidermiques et/ou empêchant leur dégradation,
- les agents anti-glycation,
- les agents anti-irritants,
- les agents hydratants,
- les agents desquamants
- les agents modifiant la pigmentation,
- les inhibiteurs de la NO-synthase,
- les agents stimulant la prolifération des fibroblastes ou des kératinocyles et/ou la différentiation des kératinocytes,
- les agents anti-pollution ou anti-radicalaire,
- les agents apaisants,
- les agents agissant sur la microcirculation,
- les agents agissant sur le métabolisme énergétique des cellules,
- les agents cicatrisants, et
- leurs mélanges.

### Actifs dermorelaxants

La composition selon l'invention peut comprendre un actif hydrophile et/ou un actif lipophile choisis parmi les agents dermorelaxants.

Les agents dermorelaxants utilisables dans la composition selon l'invention comprennent notamment l'alvérine et ses sels, les chlorures ou les gluconates de magnésium ou de manganèse, le Diazepam, l'hexapeptide Argireline R commercialisé par la société LIPOTEC, les amines secondaires et tertiaires carbonylées telles que décrites dans la demande EP-A-1405633 (notament la 3-(éthyl-(3-hydroxy-3-pentyl-octyl)-amino) propiophénone), l'adénosine, ainsi que les sapogénines et les extraits naturels, en particulier de Wild Yam, en contenant, ainsi que l'acide 3-O-acétyl 11-céto boswellique et les extraits végétaux en contenant tel que l'extrait de Boswellia serrata, comme décrit dans la demande EP-A-1442736; et leurs mélanges.

De préférence, les agents dermorelaxants sont choisis parmi le gluconate de magnésium, le gluconate de manganèse, les sapogénines extraites de Wild Yam, l'extrait de de Boswellia serrata ; et leurs mélanges.

### Les agents stimulant la synthèse de macromolécules dermiques ou épidermiques et/ou empêchant leur dégradation

La composition selon l'invention peut comprendre un actif hydrophile et/ou un actif lipophile stimulant la synthèse de macromolécules dermiques ou épidermiques et/ou empêchant leur dégradation.

De préférence, les agents stimulant la synthèse de macromolécules dermiques ou épidermiques et/ou empêchant leur dégradation sont choisis parmi les extraits de Centella asiatica, l'acide ascorbique et ses dérivés, les peptides extraits de végétaux, tels que l'hydrolysat de soja commercialisé par la société COLETICA sous la dénomination commerciale Phytokince^{®}, l'extrait de Saccharomyces Cerivisiae commercialisé par la société LSN sous la dénomination commerciale Cytovitin^{®} ; l'extrait d'algue brune Padina pavonica commercialisé par la société ALBAN MÜLLER sous la dénomination commerciale HSP3^{®} ; les rétinoïdes et dérivés ; les extraits de romarin ; l'extrait peptidique de graines de légumineuse (Pisum sativum) commercialisé par la société LSN sous la dénomination commerciale Parclastyl^{®} ; l'acide {2-[acétyl-(3-trifluorométhyl-phényl)-amino]-3-méthyl-butyrylamino} acétique ; l'extrait de lupin ; et leurs mélanges.

### Les agents anti-glycation

La composition selon l'invention peut comprendre un actif hydrophile ou un actif lipophile choisi parmi les agents anti-glycation.

Par « agent anti-glycation », on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille *(Vaccinium angusfifollium)*, par exemple celui vendu sous la dénomination « BLUEBERRY HERBASOL EXTRACT PG » par la société COSMETOCHEM; l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 2 802 425, FR 2 810 548, FR 2 796 278 et FR 2 802 420, respectivement. On peut également citer les polypeptides d'arginine et de lysine tels que celui vendu sous la dénomination « AMADORINE » par la société SOLABIA ; et leurs mélanges.

On peut utiliser comme agent anti-glycation l'extrait de myrtille.

La composition selon l'invention comprenant un agent anti-glycation tel que défini ci-dessus peut avantageusement être utilisée pour prévenir ou traiter les signes du vieillissement de la peau liés à la glycation, en particulier pour prévenir ou traiter la perte de tonicité des lèvres et du contour des lèvres due à l'âge.

### Les agents anti-irritants

La composition selon l'invention peut comprendre un actif hydrophile ou un actif lipophile choisi parmi les agents anti-irritants.

On entend par « agent anti-irritant » un actif modulant les manifestations d'irritation des lèvres ou du contour des lèvres, tels que les picotements, les tiraillements, et la sensation de chaleur.

Plus précisément, on utilise selon l'invention comme agents anti-irritants :
- des agents bloqueurs de canaux sodiques, et/ou
- des agents qui interagissent spécifiquement avec les récepteurs des neuromédiateurs ou des neurohormones, choisis parmi les antagonistes de la substance P, les antagonistes du CGRP, et les antagonistes de la bradykinine.

A titre d'exemples, sont utilisables dans l'invention les inhibiteurs de canaux sodiques comme: l'Amiloride, la Quinidine, la Quinidine sulfate, l'Apamine, la Cyproheptadine, la Loperamide et la N-acétylprocaïnamide.

Des exemples d'antagonistes de substances P utilisables dans l'invention sont notamment : les sels de strontium ; les eaux thermales et en particulier l'eau thermale du bassin de Vichy et l'eau thermale de La Roche Posay ; les extraits bactériens et en particulier l'extrait de bactéries filamenteuses non photosynthétiques décrit dans la demande de brevet EP-0 761 204, préparé de préférence à partir de bactéries appartenant à l'ordre des Beggiatoales, et plus particulièrement aux genres Beggiatoa, Vitreoscilla, Flexithrix ou Leucothrix. Préférentiellement, on utilise selon l'invention une souche de *Vitreoscilla filiformis.*

Un exemple non limitatif d'antagoniste de CGRP utilisable dans la présente invention est constitué par un extrait de cellules (de préférence indifférenciées) d'au moins un végétal de la famille des Iridacées, obtenu par culture *in vitro.* L'Iridacée appartient de préférence à l'un des genres suivants : Romulea, Crocus, Iris, Gladiolus, Sisyrinchium et Hermodactylus. Pour une utilisation dans la présente invention, on préfère utiliser un extrait de matériel végétal provenant d'Iris, mieux, d*'Iris pallida,* comme décrit dans la demande EP-0 765 668,

Un exemple non limitatif d'antagoniste de bradykinine utilisable dans la présente invention est constitué par un extrait d'au moins un végétal de la famille des Rosacées, de préférence cultivé *in vivo.* L'extrait de Rosacée peut appartenir préférentiellement aux genres suivants : Agrimonia, Amygdalus, Armeniaca, Cerasus, Malus, Mespilus, Persica, Pirus, Prunus, Rosa, Rubus.

Préférentiellement, on utilise selon l'invention un végétal appartenant au genre Rosa, avantageusement préparé à partir de matériel issu d'au moins un végétal appartenant à une espèce choisie parmi *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa, Rosa villosa.* Mieux, le végétal appartient à l'espèce *Rosa gallica* comme décrit dans la demande de brevet EP-0 909 556.

L'agent anti-irritant utilisé selon l'invention peut être d'origine naturelle ou synthétique. Par origine naturelle, on entend un inhibiteur à l'état pur ou en solution quelle qu'en soit sa concentration dans ladite solution, obtenu par différents procédés à partir d'un élément naturel. Par origine synthétique, on entend un agent anti-irritant à l'état pur ou en solution, quelle qu'en soit sa concentration dans ladite solution, obtenue par synthèse chimique.

L'agent anti-irritant peut être également choisi parmi les sels de la mer morte, les extraits de pivoine, notamment de racine de Paeonia suffruticosa, par exemple commercialisé sous la dénomination « BOTANPI LIQUID B » par la société ICHIMARU PHARCOS, la dermocalmine de chez Silab ou les extraits de graines de lin, tel que celui vendu sous la dénomination « SENSILINE » par la société Silab.

### Les agents desquamants

La composition selon l'invention peut contenir un actif hydrophile ou un actif lipophile choisi parmi les agents desquamants.

Par « agent desquamant », on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

### Les agents hydratants

La composition selon l'invention peut contenir un actif hydrophile ou un actif lipophile choisi parmi les agents hydratants.

Par « agent hydratant », on entend :
- soit un composé agissant sur la fonction barrière, en vue de maintenir l'hydratation du stratum corneum, ou un composé occlusif. On peut citer les céramides, les composés à base sphingoïde, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, la glycérine, le pentanediol, le pidolate de sodium, la sérine, le xylitol, le lactate de sodium, le polyacrylate de glycérol, l'ectoïne et ses dérivés, le chitosane, les oligo- et polysaccharides comme le produit commercialisé sous la référence Pentavitin, le miel, les alginates (notamment le produit Sobalg PH 154 commercialisé par la société Grindsted), les carbonates cycliques, l'acide N-lauroyl pyrrolidone carboxylique ou ses sels, notamment le sel de sodium commercialisé sous la référence Nalidone, et la N-α-benzoyl-L-arginine ;
- soit un composé activant les glandes sébacées tel que les dérivés stéroïdiens (dont la DHEA, ses dérivés 7-oxydés et/ou 17-alkylés et les sapogénines), le dihydrojasmonate de méthyle, et la vitamine D et ses dérivés.

### L'agent dépigmentant, anti-pigmentant ou pro-pigmentant

La composition selon l'invention peut contenir un actif hydrophile ou un actif lipophile choisis parmi les agents dépigmentants, anti-pigmentants ou pro-pigmentants.

Les agents dépigmentants ou anti-pigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.

Comme agent pro-pigmentant, on peut citer l'extrait de pimprenelle (Sanguisorba officinalis) commercialisé par la société MARUZEN et les extraits de chrysanthème (Chrysanthemum morifolium).

### Les inhibiteur de NO-synthase

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucosclect^{®}, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

### Agent stimulant la prolifération des fibroblastes ou des kératinocytes et/ou la différenciation des kératinocytes

Les agents hydrophiles ou lipophiles stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; l'adénosine ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine^{®}; le beta-sitosteryl sulfate de sodium commercialisé par la société SEPORGA sous la dénomination commerciale Phytocohésine^{®} ; et l'extrait de maïs commercialisé par la société SOLABIA sous la dénomination commerciale Phytovityl^{®} ; et les lignanes tels que le sécoisolaricirésinol.

### Agent anti-pollution ou anti-radicalaire

La composition selon la présente invention peut contenir un actif hydrophile ou un actif lipophile choisi parmi les agents anti-pollution. Par l'expression « agent anti-pollution », on entend tout composé capable de piéger l'ozone, les composés aromatiques mono- ou polycycliques tels que le benzopyrène et/ou les métaux lourds tels que le cobalt, le mercure, le cadmium et/ou le nickel. Par « agent anti-radicalaire », on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F^{®}, la fraction hydrosoluble de maïs commercialisée par la société SOLABIA sous la dénomination commerciale Phytovityl^{®}, le mélange d'extrait de fumeterre et d'extrait de citron commercialisé sous la dénomination Unicotrozon C-49^{®} par la société Induchem, et le mélange d'extraits de ginseng, de pomme, de pêche, de blé et d'orge vendu par la société PROVITAL sous la dénomination commerciale Pronalen Bioprotect^{®}.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl^{®}.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl^{®}.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase et les extraits de germes de blé en contenant, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; la guanosine ; les lignanes ; et la mélatonine.

### Agents apaisants

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, les extraits de plantes telles que *Paeonia suffruticosa* et / ou *lactiflora, Laminaria saccharina, Boswellia serrata, Centipeda cunnighami, Helianthus annuus, Linum usitatissimum, Cola nitida, Epilobium Angustifolium, Aloe vera, Bacopa monieri,* les sels de l'acide salicylique et en particulier le salicylate de zinc, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'ecchium, ou de poisson, des extraits de plancton, la capryloyl glycine, le Seppicalm VG (sodium palmitoylproline et nymphea alba) de Seppic, les tocotrienols, le piperonal, un extrait de clou de girofle, les phytostérols, la cortisone, l'hydrocortisone, l'indométhacine et la beta méthasone.

### Agents agissant sur la microcirculation

Les actifs agissant sur la microcirculation (vasoprotecteur ou vasodilatateur) peuvent être choisis parmi les flavonoïdes, les ruscogénines, les esculosides, l'escine extraite du marron d'Inde, les nicotinates, l'héperidine méthyl chalcone, les huiles essentielles de lavande ou de romarin, les extraits de Ammi Visnaga.

### Agents agissant sur le métabolisme énergétique des cellules

Les actifs concernés sont ceux qui agissent sur le métabolisme énergétique cutané tel que, par exemple, et de façon non limitative, la synthèse d'ATP, ceux qui sont interviennent sur la chaine respiratoire de la cellule ou sur les réserves énergétiques. On peut citer le Coenzyme Q10 (ubiquinone), le cytochrome C, la créatine ou encore la phosphocréatine.

### Agents cicatrisants

Des exemples d'agents cicatrisants sont notamment l'extrait de feuilles de fougère commercialisé sous la référence Mamaku Vital Essence par Lucas Meyer, les peptides de riz obtenus par hydrolyse de protéines de riz commercialisés sous la dénomination Nutripeptide par Silab.

Parmi les actifs hydrophiles et les actifs lipophiles pouvant entrer dans la composition selon l'invention on peut encore citer les actifs choisis parmi les caroténoides (l'alpha-carotène, le bêta-carotène,; la zéaxanthine ; la lutéine ; l'ataxanthine, la bixine), la canthaxantine, la crypthoxanthine, les rétinoïdes (le rétinol, le rétinal , l'acide rétinoïque 13-cis, l'acide rétinoïque all-trans), les flavanones (naringénine et hespérétine, le glycosyle hespérétine), les flavonols (Kaempférol, quercétol), les isoflavones (isoflavone, daidzéine, génistéine), les coumarines (l'esculoside, la visnadine , l'esculetol ,le 4-methylesculetol, le PERMETHOL de SYPHETAL tel que le sel de sodium de l'acétate de méthylesculétin), les lignanes (le silymarine, l'acide nordihydroguaïarétique , le sécoisolaricinésinol), les stilbenoides (le resvératrol et ses dérivés alcoxylés et glycosylés), les sapogénines (l'héderagénine, la diosgénine, l'hécogénine, la smilagénine), les acides triterpéniques pentacycliques (l'acide glycyrrhétinique, l'acide glycyrrhizique, l'acide ursolique, l'acide bétulinique, l'acide oléanique, l'acide asiatique, l'acide madécassique), les stérols (tels que le cholestérol, le fucostérol), les hydroxyphéols et leur derivés (l'hydroquinone, l'arbutine, l'acide homogentisique, l'acide gentisique, le catéchol, le guaïacol, le résorcinol, le lucinol, le méquinol), les acides phénoliques (l'acide cinnamique, l'acide coumarinique, l'acide cafféique, l'acide rosmarinique, l'acide ferrulique, l'acide chlorogénique), les monomères précurseurs des tanins (l'acide gallique, l'acide élagique, les extraits d'hamamélis), les aminosucres (la N-acétyl -glucosamine, la N-acétyl - galactosamine), les vitamines choisie parmi la vitamine A, la vitamine C, la vitamine E, la vitamine B3, la vitamine B5, la vitamine D, la vitamine F, et leurs dérivés, analogues et précurseurs, et leurs mélanges.

La quantité d'actif(s) va par exemple de 0,001 à 30 % en poids et de préférence de 0,01 à 20 % en poids de matière active par rapport au poids total de la composition.

### Milieu Physiologiquement acceptable

La composition de l'invention doit être cosmétiquement ou dermatologiquement acceptable, à savoir contenir un milieu physiologiquement acceptable non toxique et susceptible d'être appliqué sur les lèvres d'êtres humains. Par cosmétiquement acceptable, on entend au sens de l'invention une composition d'aspect, d'odeur et de toucher agréables.

### Phase grasse

Le milieu physiologiquement acceptable comprend avantageusement une phase grasse.

La phase grasse de la composition selon l'invention est en particulier une phase grasse liquide à base d'au moins une huile.

### Phase grasse liquide

L'huile peut être une huile siliconée, une huile ester ou une huile non siliconée.

### a. Huile siliconée

Selon une variante de l'invention, la phase grasse liquide comprend au moins une huile siliconée volatile.

Au sens de l'invention, une huile volatile présente à température ambiante (25 °C) et pression atmosphérique (760 mm de Hg) une pression de vapeur allant de 0,02 mm à 300 mm de Hg (2,66 Pa à 40 000 Pa) et mieux allant de 0,1 à 90 mm de Hg (13 Pa à 12 000 Pa). Les huiles non volatiles correspondent alors à une pression de vapeur inférieure à 0,02 mm de Hg (2,66 Pa).

L'huile siliconée volatile peut être choisie parmi les huiles siliconées linéaires ou cycliques telles que les polydiméthylsiloxanes (PDMS) linéaires ou cycliques ayant de 3 à 7 atomes de silicium.

A titre d'exemple de telles huiles, on peut citer l'octyltriméthicone, l'hexyltriméthicone, la décaméthylcyclopentasiloxane (cyclopentasiloxane ou D5), l'octaméthylcyclotétrasiloxane (cyclotétradiméthylsiloxane ou D4), la dodécaméthylcyclohexasiloxane (D6), la décaméthyltétrasiloxane (L4), KF 96 A de Shin Etsu, les polydiméthysiloxanes telles que celles commercialisées sous la référence DC 200 (1,5 cSt), DC 200 (5 cSt), DC 200 (3 cSt) par Dow Corning.

Les huiles siliconées non volatiles peuvent être des polydiméthylsiloxanes, des polyalkylméthylsiloxanes, des diméthicone copolyols, des alkylméthicone copolyols, la cétyldiméthicone, des silicones à groupes alkylglycéryl éthers, des silicones à groupes amines latéraux et le dilauroyltriméthylol propane siloxysilicate. Les groupements alkyle de ces huiles ont notamment de 2 à 24 atomes de carbone.

Les huiles siliconées non volatiles utilisables dans la phase grasse liquide peuvent être en particulier les polydiméthylsiloxanes (PDMS) non volatils, linéaires, liquides à température ambiante ; les polydiméthylsiloxanes comportant des groupements alkyle, alcoxy ou phényle, pendants et/ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, les silicones fluorées à groupement(s) pendant(s) ou en bout de chaîne ayant de 1 à 12 atomes de carbone dont tout ou partie des atomes d'hydrogène est substitué par des atomes de fluor, les diméthiconols et leurs mélanges.

Les huiles siliconées ont une viscosité choisie avantageusement dans la gamme allant de 5 à 800 000 cSt à 25 °C, de préférence de 10 à 500 000 cSt, et mieux de 10 à 5 000 cSt.

La phase grasse liquide contient avantageusement de 0,1 à 60 %, par exemple de 5 à 50 % en poids d'huile(s) siliconée(s).

### b. Huile ester

Selon une variante de l'invention, au moins une des huiles de la phase grasse liquide est une huile, dite « huile ester », qui est choisie parmi les esters des acides monocarboxyliques avec les monoalcools et polyalcools.

Avantageusement, ledit ester répond à la formule suivante :

R₁-CO-O-R₂

où R₁ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.

R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par « éventuellement substitué », on entend que R₁ et ou R₂ peuvent porter un ou plusieurs substituants choisis, par exemple, parmi les groupements comprenant un ou plusieurs hétéroatomes choisi parmi O, N et S, tels que amino, amine, alcoxy, hydroxyle.

De préférence, le nombre total d'atomes de carbone de R₁ + R₂ est ≥ 9.

R₁ peut représenter le reste d'un acide gras, de préférence supérieur, linéaire ou de préférence ramifié comprenant de 1 à 40 et mieux de 7 à 19 atomes de carbone et R₂ peut représenter une chaîne hydrocarbonée linéaire ou de préférence ramifiée contenant de 1 à 40, de préférence de 3 à 30 et mieux de 3 à 20, (19 à 28, 8 à 27, 7 à 26 C) atomes de carbone. De nouveau, de préférence, le nombre d'atomes de carbone de R₁ + R₂ ≥ 9.

Des exemples des groupes R₁ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.

Des exemples d'esters utilisables dans les phases grasses des compositions de l'invention sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

Avantageusement, les esters sont choisis parmi les composés de la formule (I) ci-dessus, dans laquelle R₁ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et R₂ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.

De préférence, R₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone. De préférence dans la formule (I), R₁-CO- et R₂ ont le même nombre d'atomes de carbone et dérivent du même radical, de préférence alkyle ramifié non substitué, par exemple isononyle, c'est-à-dire que avantageusement la molécule d'huile ester est symétrique.

L'huile ester sera choisie, de préférence, parmi les composés suivants :
- l'isononanoate d'isononyle,
- l'octanoate de cétostéaryle,
- le myristate d'isopropyle,
- le palmitate d'éthyl-2-hexyle,
- le stéarate d'octyl 2-dodécyle,
- l'érucate d'octyl 2-dodécyle,
- l'isostéarate d'isostéaryle.

L'ester préféré entre tous est l'isononanoate d'isononyle.

Selon un mode de réalisation, la composition comprend moins de 10 % d'huile ester volatile, de préférence moins de 5 % d'huile ester volatile, mieux moins de 3 % d'huile ester volatile, voire est exempte d'huile ester volatile.

Avantageusement, la phase grasse liquide comprend de 0,1 à 60 % en poids de l'huile ou des huiles esters, de préférence de 5 à 50 % en poids.

### c. Huile non siliconée

La phase grasse liquide des compositions selon l'invention peut contenir en outre une ou plusieurs huiles non siliconées volatiles ou non. Les huiles non siliconées volatiles peuvent être choisies dans le groupe des huiles hydrocarbonées et des esters et éthers volatils tels que les hydrocarbures volatils comme l'isododécane et l'isohexadécane, les isoparaffines en C₈-C₁₆.

L'huile non siliconée volatile peut aussi être choisie parmi les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés.

A titre d'exemple d'huiles non siliconées volatiles utilisables dans la composition de l'invention, on peut citer l'isododécane, l'isohexadécane, le n-butyléther de propylène glycol, le 3-éthoxypropionate d'éthyle, l'acétate de méthyléther de propylène glycol, les isoparaffines C₁₁-C₁₃ telle que l'Isopar L^{®} ou les isoparaffines en C₁₁-C₁₂ telles que Isopar H^{®}.

Lorsque la phase grasse comprend une huile volatile non siliconée, celle-ci représente avantageusement de 0,1 à 60 %, et mieux de 5 à 20 %, du poids total de la composition.

La phase grasse liquide peut aussi contenir d'autres huiles non siliconées, par exemple des huiles polaires telles que :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearines Dubois ou ceux vendus sous les dénominations Miglyol 810, 812 et 818 par la société Dynamit Nobel,
- les éthers de synthèse ayant de 10 à 40 atomes de carbone,
- les alcools gras en C₈ à C₂₆ comme l'alcool oléique ou l'octyldodécanol,
- les acides gras comme l'acide oléique, linoléique ou linolénique, et
- leurs mélanges.

La phase grasse liquide peut encore contenir des huiles apolaires telles que les hydrocarbures ou fluorocarbures, linéaires ou ramifiés, d'origine synthétique ou minérale, volatils ou non, comme les huiles de paraffine volatiles (telles que les isoparaffines, l'isododécane) ou non volatiles et ses dérivés, la vaseline, les polydécènes, le polyisobutène hydrogéné tel que le parléam, le squalane, et leurs mélanges.

La ou les huiles peuvent être présentes dans la composition selon l'invention en une teneur allant de 0,1 % à 90 % en poids, de préférence de 5 % à 80 % en poids par rapport au poids total de la composition.

### Agent structurant

La composition selon l'invention peut comprendre, outre le ou les polymère(s) siliconé(s) décrits plus haut, un agent structurant choisi parmi les cires, les polymères semi-cristallin, les gélifiants lipophiles et leurs mélanges.

Il est entendu que la quantité en ces composés annexes peut être ajustée par l'homme du métier de manière à ne pas porter préjudice à l'effet recherché dans le cadre de la présente invention.

### Cire(s)

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

Les cires pouvant être utilisées dans les compositions selon l'invention présentent généralement une dureté allant de 0,01 MPa à 15 MPa, notamment supérieure à 0,05 MPa et en particulier supérieure à 0,1 MPa.

A titre illustratif des cires convenant à l'invention, on peut notamment citer les cires hydrocarbonées comme la cire d'abeille, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50^{®}, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S^{®} par la société HETERENE.

On peut encore citer les cires de silicone (C₃₀₋₄₅ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64^{®} et 22L73^{®} par la société SOPHIM. De telles cires sont décrites dans la demande FR-A- 2792190.

Comme cire, on peut utiliser un (hydroxystéaryloxy)stéarate d'alkyle en C₂₀-C₄₀ (le groupe alkyle comprenant de 20 à 40 atomes de carbone), seul ou en mélange.

Une telle cire est notamment vendue sous les dénominations « Kester Wax K 82 P^{®} », « Hydroxypolyester K 82 P^{®} » et « Kester Wax K 80 P^{®} » par la société KOSTER KEUNEN.

Comme micro cires pouvant être utilisées dans les compositions selon l'invention, on peut citer notamment les micro cires de carnauba telles que celle commercialisée sous la dénomination de MicroCare 350^{®} par la société MICRO POWDERS, les micro cires de cire synthétique telles que celle commercialisée sous la dénomination de MicroEase 114S^{®} par la société MICRO POWDERS, les micro cires constituées d'un mélange de cire de carnauba et de cire de polyéthylène telles que celles commercialisées sous les dénominations de Micro Care 300^{®} et 310^{®} par la société MICRO POWDERS, les micro cires constituées d'un mélange de cire de carnauba et de cire synthétique telles que celle commercialisée sous la dénomination Micro Care 325^{®} par la société MICRO POWDERS, les micro cires de polyéthylène telles que celles commercialisées sous les dénominations de Micropoly 200^{®}, 220^{®}, 220L^{®} et 250S^{®} par la société MICRO POWDERS et les micro cires de polytétrafluoroéthylène telles que celles commercialisées sous les dénominations de Microslip 519^{®} et 519 L^{®} par la société MICRO POWDERS.

La composition selon l'invention peut comprendre une teneur en cires allant de 0,1 à 30 % en poids par rapport au poids total de la composition, en particulier elle peut en contenir de 0,5 à 15 %, plus particulièrement de 1 à 10 %.

### Composés pâteux

La composition selon l'invention peut comprendre en outre un composé pâteux qui peut être avantageusement choisi parmi :
- la lanoline et ses dérivés
- les composés siliconés polymères ou non
- les composés fluorés polymères ou non
- les polymères vinyliques, notamment :
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
- et leurs mélanges.

Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique,
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H^{®}, et Risocast DA-L^{®},
- et leurs mélanges.

Parmi les composés pâteux d'origine végétale, on choisira de préférence un mélange de stérols de soja et de pentaérythritol oxyéthyléné (SOE) oxypropyléné (5 OP), commercialisé sous la référence Lanolide par la société VEVY.

Selon un mode de réalisation, la composition comprend moins de 10 % en poids, de préférence moins de 7 %, mieux moins de 5 %, et encore mieux moins de 3 % en poids de cire par rapport au poids total de la composition. De préférence encore, la composition est totalement exempte de cire.

### Gélifiants lipophiles

Les gélifiants utilisables dans les compositions selon l'invention peuvent être des gélifiants lipophiles organiques ou minéraux, polymériques ou moléculaires. Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium telle que, par exemple, celle commercialisée sous la dénomination de Bentone 38V^{®} par la société ELEMENTIS.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6^{ème} édition, 1995). Elles sont par exemple commercialisées sous les références Aerosil R812^{®} par la société DEGUSSA, CAB-O-SIL TS-530^{®} par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6^{ème} édition, 1995). Elles sont par exemple commercialisées sous les références Aerosil R972^{®}, et Aerosil R974^{®} par la société DEGUSSA, CAB-O-SIL TS-610^{®} et CAB-O-SIL TS-720^{®} par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6^{®}, KSG16^{®} et de KSG18^{®} par la société SHIN-ETSU, de Trefil E-505C^{®} et Trefil E-506C^{®} par la société DOW-CORNING, de Gransil SR-CYC^{®}, SR DMF10^{®}, SR-DC556^{®}, SR 5CYC gel^{®}, SR DMF 10 gel^{®} et de SR DC 556 gel^{®} par la société GRANT INDUSTRIES, de SF 1204^{®} et de JK 113^{®} par la société GENERAL ELECTRIC ; l'éthylcellulose comme celle vendue sous la dénomination Ethocel^{®} par la société DOW CHEMICAL ; les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG^{®} par la société ARIZONA CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB^{®} par la société BASF, du type polystyrène/copoly(éthylène-propylène) tels que ceux commercialisés sous la dénomination de Kraton^{®} par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel^{®} comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL^{®} ou Rheopearl KL^{®} par la société CHIBA FLOUR.

### Phase aqueuse

Le milieu physiologiquement acceptable de la composition selon l'invention peut comprendre un milieu aqueux, constituant une phase aqueuse, qui peut former la phase continue de la composition.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄ et leur mélanges.

La phase aqueuse (eau et éventuellement le solvant miscible à l'eau) peut être présente, en une teneur allant de 1 % à 95 % en poids, par rapport au poids total de la composition, de préférence allant de 2 % à 80 % en poids, et préférentiellement allant de 3 % à 60 % en poids.

La composition selon l'invention peut également contenir moins de 10 % en poids voire moins de 4 % en poids de phase aqueuse ou d'eau.

Selon un mode de réalisation préféré, la composition est anhydre.

### Système émulsionnant

Outre le tensioactif phosphaté, les compositions selon l'invention peuvent en outre contenir un ou plusieurs tensioactifs.

Selon une variante de l'invention, elles comprennent au moins un tensioactif siliconé.

Le ou les tensio-actifs siliconé(s) peut être présent dans la composition en une teneur allant de 0,1 % à 50 % en poids, par rapport au poids total de la composition, en particulier allant de 0,1 % à 40 % en poids, plus particulièrement allant de 0,5 % à 30 % en poids, en particulier allant de 0,5 % à 20 % en poids, et encore plus particulièrement allant de 1 % à 10 % en poids.

Parmi les agents tensioactifs utilisables dans les compositions cosmétiques conformes à la présente invention, on peut mentionner plus particulièrement les organopolysiloxanes hydrophiles différents du polymère siliconé décrit précédemment.

Le radical hydrophile peut répondre à la formule : 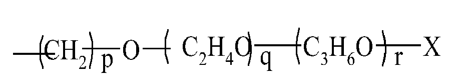 dans laquelle
- p varie de 0 à 5, q varie de 0 à 100, r varie de 0 à 50, avec p ou q étant différent de zéro,
- les unités (C₂H₄ O) et (C₃ H₆ O) peuvent être réparties aléatoirement ou par blocs, et
- X est un hydrogène ou un radical alkyle en C₁-C₁₀ le cas échéant substitué par un ou plusieurs fonctions de type hydroxyle, thiol, amine, carboxylique, carboxylate, amide, phosphate, sulfate et sulfonate.
   En particulier, p peut varier de 1 à 5, q de 1 à 100 et v de 1 à 50. X peut plus particulièrement figurer un atome d'hydrogène.
   En particulier, l'organopolysiloxane, selon l'invention, peut comprendre à titre de radical hydrophile au moins un radical hydroxy-polyalkylèneoxy et notamment un radical hydroxy-polyéthylèneoxy.
   Notamment, l'organopolysiloxane selon l'invention peut répondre à la formule : 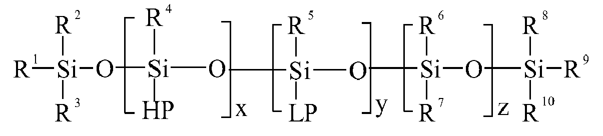
dans laquelle
- R¹, R², R³, R⁴, R⁵, R⁶ R⁷, R⁸, R⁹ et R¹⁰ représentent indépendamment l'un de l'autre un radical alkyle en C₁ à C₆, linéaire, ramifié ou cyclique, saturé ou non,
- HP est un radical portant au moins un groupement hydrophile tel que défini précédemment,
- LP est un radical lipophile, et
- x varie de 1 à 5000 ; y de 0 à 5000 ; z de 0 à 5000.

En ce qui concerne le radical LP, il peut notamment être choisi parmi des alkyles en C₁ à C₄₀ ramifiés, cycliques ou linéaires, des groupements organosiloxanes, des atomes de fluors, des radicaux aryles, aryloxy, des acyles d'hydrocarbyles en C₁ à C₄₀ et des hydroxypropylénoxy.

Selon une variante particulière de l'invention, l'organopolysiloxane appartient à la famille des diméthicones copolyol, en particulier la cétyldiméthicone copolyol et leurs dérivés. L'organopolysiloxane hydrophile selon la présente invention peut être le produit commercialisé sous la marque Abil WE09 ou Abil EM90 par la société Degussa-Goldschmidt. L'organopolysiloxane hydrophile selon la présente invention peut être également le produit commercialisé sous la référence KF-6017 par la société Shin-Etsu.

Le composé organopolysiloxane peut être en tout ou partie fluoré. En particulier, les groupements di-alkyles inférieurs siloxy peuvent être substitués par un ou plusieurs atomes de fluor.

Selon un mode particulier de réalisation, l'agent tensioactif siliconé utilisable dans les compositions cosmétiques conformes à la présente invention est choisi parmi la diméthicone copolyol, la diméthicone copolyol benzoate, les diméthicones copolyols phosphates, les élastomères de silicone polyoxyalkylénés, le mélange cyclométhicone/diméthicone et leurs mélanges.

Conviennent également selon l'invention les élastomères de silicones polyoxyalkylénés tels que ceux décrits dans les brevets US5236986, US5412004, US5837793, US5811487 dont le contenu est incorporé par référence.

Comme élastomère de silicone polyoxyalkyléné, on peut utiliser ceux commercialisés sous les dénominations « KSG-21 », « KSG-20 », « KSG-30 », « KSG-31 », « KSG-32 », « KSG-33 », « KSG-210 », « KSG-310 », « KSG-320 », « KSG-330 », « KSG-340 », « X-226146 » par la société SHIN ETSU, « DC9010 », « DC9011 » par la société DOW CORNING.

Il est entendu que les compositions selon l'invention peuvent en outre comprendre des agents tensioactifs non siliconés anioniques et/ou non ioniques.

Pour le choix de ces agents tensioactifs, on peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs pouvant être plus particulièrement utilisés dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges,
- parmi les tensioactifs anioniques : les acides gras en C₁₆-C₃₀ neutralisés par les amines, l'ammoniaque ou les sels alcalins, et leurs mélanges.

### Polymère filmogène

La composition selon l'invention peut comprendre selon un mode de réalisation particulier au moins un polymère filmogène.

Le polymère filmogène peut être présent dans la composition selon l'invention en une teneur en matières sèches (ou matières actives) allant de 0,1 % à 30 % en poids par rapport au poids total de la composition, de préférence de 0,5 % à 20 % en poids, et mieux de 1 % à 15 % en poids.

Dans la présente invention, on entend par « polymère filmogène », un polymère apte à former à lui seul ou en présence d'un agent auxiliaire de filmification, un film macroscopiquement continu par exemple lorsque ledit film est réalisé par coulage sur une surface antiadhérente comme une surface téflonnée ou siliconnée. Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆ .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être un polymère hydrosoluble et peut être présent dans une phase aqueuse de la composition ; le polymère est donc solubilisé dans la phase aqueuse de la composition.

Selon une autre variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2 000 à 500 000 et de préférence de 4 000 à 200 000.

Comme polymères filmogènes liposolubles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de « MDTQ », la résine étant décrite en fonction des différentes unités monomèriques siloxane qu'elle comprend, chacune des lettres « MDTQ » caractérisant un type d'unité.

A titre d'exemples de résines polymethylsilsesquioxanes commercialement disponibles, on peut citer celles qui sont commercialisés :
- par la société Wacker sous la référence Resin MK tels que la Belsil PMS MK, et
- par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination « KF-7312J » par la société Shin-Etsu, « DC 749 », « DC 593 » par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'un résine de silicone, telle que celles décrite plus haut, et d'un diorganosiloxane tels que décrits dans le document WO 2004/073626.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1 411 069 ou WO 04/028488.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90^{®}, Neocryl A-1070^{®}, Neocryl A-1090^{®}, Neocryl BT-62^{®}, Neocryl A-1079^{®} et Neocryl A-523^{®} par la société AVECIA-NEORESINS, Dow Latex 432^{®} par la société DOW CHEMICAL, Daitosol 5000 AD^{®} ou Daitosol 5000 SJ^{®} par la société DAITO KASEY KOGYO; Syntran 5760^{®} par la société Interpolymer, Allianz OPT par la société ROHM & HAAS, les dispersions aqueuses de polymères acryliques ou styrène/acrylique vendues sous le nom de marque JONCRYL^{®} par la société JOHNSON POLYMER ou encore les dispersions aqueuses de polyuréthane vendues sous les dénominations Neorez R-981^{®} et Neorez R-974^{®} par la société AVECIA-NEORESINS, les Avalure UR-405^{®}, Avalure UR-410^{®}, Avalure UR-425^{®}, Avalure UR-450^{®}, Sancure 875^{®}, Sancure 861^{®}, Sancure 878^{®} et Sancure 2060^{®} par la société GOODRICH, Impranil 85^{®} par la société BAYER, Aquamere H-1511^{®} par la société HYDROMER ; les sulfopolyesters vendus sous le nom de marque Eastman AQ^{®} par la société Eastman Chemical Products, les dispersions vinyliques comme le Mexomère PAM^{®} de la société CHIMEX et leurs mélanges.

Comme exemples de dispersions non aqueuses de polymère filmogène, on peut citer les dispersions acryliques dans l'isododécane comme le Mexomère PAP^{®} de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

### Matières colorantes

Selon un mode de réalisation, la composition selon l'invention peut contenir en outre au moins une matière colorante, organique ou inorganique, notamment de type pigments ou nacres.

Selon un autre mode de réalisation, la composition selon l'invention peut contenir en outre au moins une matière colorante choisie parmi les colorants lipophiles, les colorants hydrophiles, les pigments, les nacres, les matériaux à effet optique spécifique, et leurs mélanges.

Cette matière colorante peut être présente à raison de 0,01 à 50 % en poids par rapport au poids total de la composition, en particulier de 0,5 à 40 % et plus particulièrement de 5 à 25 %, et notamment de 0,01 à 20 %, et en particulier de 0,1 à 10 %, voire de 2 à 5 % en poids par rapport au poids total de la composition.

Par « pigments », il faut comprendre des particules blanches ou colorées, minérales ou organiques, insolubles dans une solution aqueuse, destinées à colorer et/ou opacifier le film résultant.

Les pigments peuvent être présents à raison de 0,01 à 20 % en poids, notamment de 0,01 à 5 % en poids, et en particulier de 0,02 à 7 % en poids, par rapport au poids total de la composition cosmétique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF^{®} NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL^{®} PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA^{®}, FLAMENCO^{®} et DUOCHROME^{®} (sur base de mica) commercialisées par la société ENGELHARD, les nacres TIMIRON^{®} commercialisées par la société MERCK, les nacres sur base de mica PRESTIGE^{®} commercialisées par la société ECKART et les nacres sur base de mica synthétique SUNSHINE^{®} commercialisées par la société SUN CHEMICAL.

Les nacres peuvent plus particulièrement posséder une couleur ou un reflet jaune, rose, rouge, bronze, orangé, brun, or et/ou cuivré.

Les pigments peuvent être ou non enrobés superficiellement, en particulier traités en surface par des silicones, des acides aminés, des dérivés fluorés ou toute autre substance favorisant la dispersion et la compatibilité du pigment dans la composition.

Avantageusement, les pigments utilisés dans les compositions conformes à l'invention peuvent également être enrobés superficiellement par un revêtement de lécithine. Ce revêtement peut être obtenu par mise en contact d'une solution de pigment avec une solution de lécithine, en présence de sels de métaux di- ou trivalents. Pour obtenir ce revêtement, de la lécithine hydrogénée ou non hydrogénée peut être utilisée.

La composition cosmétique selon l'invention peut comporter également des colorants hydrosolubles ou liposolubles en une teneur allant de 0,01 à 10 % en poids, notamment allant de 0,01 à 5 % en poids par rapport au poids total de la composition cosmétique.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Lorsque les compositions cosmétiques selon l'invention comportent un colorant hydrosoluble, ce dernier peut être présent dans la composition à l'état dispersé.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on entend désigner des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC^{®} par la société SIBERLINE et METALURE^{®} par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE^{®} 700 de la société ECKART, les particules d'aluminium enrobé de silice commercialisées sous la dénomination VISIONAIRE BRIGHT SILVER^{®} de la société ECKART et les particules d'alliage métallique comme des poudres de bronze (alliage cuivre et zinc) enrobé de silice commercialisées sous la dénomination de VISIONAIRE BRIGHT NATURAL GOLD^{®} de la société ECKART.

Il peut encore s'agir de particules comportant un substrat de verre comme celles commercialisées par la société NIPPON SHEET GLASS sous les dénominations MICROGLASS METASHINE^{®}.

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂ ; SnO/TiO₂/SiO₂/TiO₂/SnO; Fe₂O₃/SiO₂/Fe₂O₃ ; SnO/mica/TiO₂/SiO₂/TiO₂/mica/SnO, des pigments ayant ces structures étant commercialisés sous la dénomination XIRONA^{®} par la société MERCK (Darmstadt). A titre d'exemple, ces pigments peuvent être les pigments de structure silice/oxyde de titane/oxyde d'étain commercialisés sous le nom XIRONA MAGIC^{®} par la société MERCK, les pigments de structure silice/oxyde de fer brun commercialisés sous le nom XIRONA INDIAN SUMMER^{®} par la société MERCK et les pigments de structure silice/oxyde de titane/mica/oxyde d'étain commercialisés sous le nom XIRONA CARRIBEAN BLUE^{®} par la société MERCK. On peut encore citer les pigments INFINITE COLORS^{®} de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE^{®} HC par la société WACKER.

La composition de l'invention peut comprendre, en outre, tout ingrédient usuellement utilisé dans le domaine concerné.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

La composition de l'invention peut se présenter sous la forme d'un produit coloré de maquillage des lèvres comme un rouge à lèvres, un brillant à lèvres ou un crayon, présentant éventuellement des propriétés de soin ou de traitement. Elle peut se présenter sous la forme d'un stick anhydre.

La composition selon l'invention peut être fabriquée par les procédés connus, généralement utilisés dans le domaine cosmétique ou dermatologique. Par exemple, elle peut être fabriquée par le procédé suivant.

Dans un premier temps, les charges et les pigments peuvent être broyés dans une partie de la phase huileuse.

Le reste des ingrédients liposolubles et le ou les tensioactif(s) phosphaté(s) peuvent être ensuite mélangés à une température de l'ordre de 100 °C. Le broyat ou les actifs pré-dispersés peuvent alors être ajoutés dans la phase huileuse.

Les éventuels actifs hydrophiles peuvent ensuite être dispersés à l'aide d'un agitateur mécanique.

Enfin, la composition peut être coulée dans un moule apte à lui procurer la forme de dôme et le tout peut être laissé refroidir à température ambiante.

Selon un autre aspect, l'invention concerne également un ensemble cosmétique comprenant :
i) un récipient délimitant un compartiment, ledit récipient étant fermé par un élément de fermeture ; et
ii) une composition conforme à l'invention disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, tel qu'un clapet par exemple.

Les exemples suivants sont donnés à titre illustratif et non limitatif de la présente invention.

### Exemples 1 à 3 de rouges à lèvres

Les compositions des exemples 1 à 3 sont obtenues selon le protocole suivant.

Dans un premier temps, les charges et les pigments sont broyés dans une partie de la phase huileuse.

Le reste des ingrédients liposolubles et le tensioactif (cetyl PEG/PGG-10/1 diméthicone, phosphate de trioleyle ou lécithine de soja) sont ensuite mélangés à une température de l'ordre de 100 °C. Le broyat ou les actifs pré-dispersés sont alors ajoutés dans la phase huileuse.

Les actifs hydrophiles sont ensuite dispersés à l'aide d'un agitateur Moritz.

Enfin, la composition est coulée dans un moule pour lui donner la forme de dôme et le tout est laissé refroidir à température ambiante.

| | Exemple 1 (comparatif) | Exemple 2 | Exemple 3 |
|---|---|---|---|
| Nom | concentration (% massique) | concentration (% massique) | concentration (% massique) |
| Polyamide/polydiméthysiloxane¹ | 25 | 25 | 25 |
| Polyamide/polydiméthysiloxane² | 1,2 | 1,2 | 1,2 |
| Phosphate de trioleyle³ | | 3 | |
| Cethyl PEG/PGG-10/1 Dimethicone⁴ | 3 | | |
| Lécithine de soja⁵ | - | - | 3 |
| Phenyltrimethicone⁶ | 37,9 | 37,9 | 37,9 |
| Polydimethylsiloxane⁷ | 11,58 | 11,58 | 11,58 |
| octyldodécanol | 0,5 | 0,5 | 0,5 |
| Isoparaffine (6-8 moles d'isobutylène) hydrogénée⁸ | 15,68 | 15,68 | 15,68 |
| Oxyde de titane | 0,57 | 0,57 | 0,57 |
| D&C RED 7 | 1,1 | 1,1 | 1,1 |
| Oxyde de fer noir | 0,47 | 0,47 | 0,47 |
| Oxydes de fer brun, jaune | 1 | 1 | 1 |
| Lauroyl Lysine | 2 | 2 | 2 |

| | | | |
|---|---|---|---|
| ¹ DC 2-8179 de Dow Corning ² DC 2-8178 de Dow Corning ³ NIKKOL TOP-OV de NIKKO ⁴ ABIL EM 90 de GOLDSCHMIDT ⁵ TOPCITHIN 300 de DEGUSSA ⁶ DC 556 de Dow Corning ⁷ DC 200 Fluid (10Cst) de Dow Corning ⁸ Parléam de NOF | | | |

### Comparaison de l'évaluation sensorielle et de l'évaluation instrumentale des compositions des exemples 1 à 3

Pour l'évaluation sensorielle, chaque composition est appliquée sur le dos de la main de cinq sujets. Les sujets évaluent alors les caractéristiques d'application et les caractéristiques du résultat du maquillage selon une échelle de 0 à 10, avec 0 représentant l'absence de la caractéristique et 10 l'évaluation maximale.

La valeur du paramètre attribué à chaque caractéristique correspond à la moyenne des valeurs données pour chacun des cinq sujets.

Pour l'évaluation instrumentale, les mesures sont réalisées au TA-XT2i^{®} selon les méthodes de mesure décrites précédemment.

| | Exemple 1 (comparatif) | Exemple 2 | Exemple 3 |
|---|---|---|---|
| EVALUATION SENSORIELLE | | | |
| A L'APPLICATION | | | |
| Glissant | 2,6 | 6,6 | 4,6 |
| Collant | 7,0 | 3,2 | 4,0 |
| Douceur | 3,6 | 6,0 | 6,0 |
| Elasticité | 3,8 | 6,0 | 5,6 |
| RESULTAT MAQUILLAGE | | | |
| Homogénéité du film | 3,4 | 6,8 | 6,4 |
| Brillance | 4,2 | 7,0 | 7,0 |
| Couvrance | 3,6 | 5,2 | 6,0 |

| EVALUATION INSTRUMENTALE | | | |
|---|---|---|---|
| Dureté (en g) | 166 | 193 | 151 |
| Elasticité (en %) | 97 | 96 | 95 |

On constate que les compositions selon l'invention des exemples 2 et 3 présentent des qualités d'application et un résultat de maquillage améliorés par rapport à l'exemple comparatif 1 tout en conservant une dureté et une élasticité comparable ou supérieure à celle de l'exemple 1.

### Exemple 4 : Composition de soin pour les lèvres

| | Concentration % massique |
|---|---|
| Polyamide/polydiméthysiloxane¹ | 25 |
| Polyamide/polydiméthysiloxane² | 1,2 |
| Phenyltrimethicone³ | 34,72 |
| Isoparaffine (6-8 moles d'isobutylène) hydrogénée⁴ | 14,46 |
| Phosphate de trioleyle⁵ | 3 |
| Glycérine | 7,5 |
| Lauryol lysine | 2 |
| Polydimethylsiloxane (10cst)⁶ | 12,12 |

| | |
|---|---|
| ¹DC 2-8179 de Dow Corning ² DC 2-8178 de Dow Corning ³ DC 556 de Dow Corning ⁴ Parléam de NOF ⁵ NIKKOL TOP-0V de NIKKO ⁶ DC 200 de Dow Corning | |

Cette composition peut aussi être pigmentée et contenir du saccharinate de sodium.

Cette composition conduit après application sur les lèvres à un dépôt brillant et homogène. L'application est agréable. La dureté de cette composition est de 189 g et l'élasticité de 95 %.

## Revendications

1. Composition cosmétique pour les lèvres comprenant dans un milieu physiologiquement acceptable au moins :
- un tensioactif phosphaté, et
- un polymère siliconé comportant au moins un motif comprenant :
(1) des polyorganosiloxanes comportant au moins deux groupes capables d'établir des interactions hydrogène, ces deux groupes étant situés dans la chaîne du polymère, et/ou
(2) des polyorganosiloxanes comportant au moins deux groupes capables d'établir des interactions hydrogène, ces deux groupes étant situés sur des greffons ou ramifications.

2. Composition selon la revendication précédente à l'état solide.

3. Composition selon la revendication 1 ou 2 présentant une dureté variant de 10 à 250 g et/ou une élasticité supérieure à 80 %.

4. Composition selon l'une quelconque des revendications précédentes ayant une dureté variant de 10 à 250 g, voire de 30 à 200 g, notamment de 50 à 190 g.

5. Composition selon l'une quelconque des revendications précédentes ayant une élasticité supérieure à 80 %, voire supérieure à 90 % et en particulier proche de 95 %.

6. Composition selon l'une quelconque des revendications précédentes, ayant une teneur totale en polymères siliconés représentant de 0,5 à 70 %, de préférence de 5 à 50 % et mieux encore de 10 à 45 % du poids total de la composition.

7. Composition selon l'une des revendications précédentes, dans laquelle lesdits motifs capables d'établir des interactions hydrogène sont choisis parmi les groupes ester, amide, sulfonamide, carbamate, thiocarbamate, urée, uréthane, thiourée, oxamido, guanidino, biguanidino et leurs combinaisons.

8. Composition selon l'une quelconque des revendications précédentes, dans laquelle le polymère siliconé comprend au moins un motif répondant à la formule générale I: 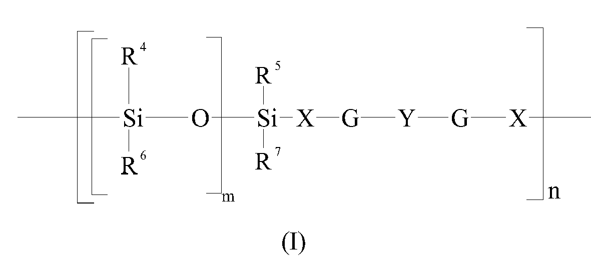 dans laquelle :
1) R⁴, R⁵, R⁶ et R⁷, identiques ou différents, représentent un groupe choisi parmi :
- les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en C₆ à C₁₀, éventuellement substitués par un ou plusieurs groupes alkyle en C₁ à C₄,
- les chaînes polyorganosiloxanes contenant ou non un ou plusieurs atomes d'oxygène, de soufre et/ou d'azote,
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 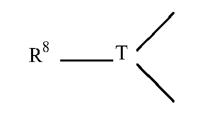 dans laquelle
- T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et A1, et
- R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi : 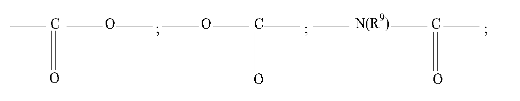 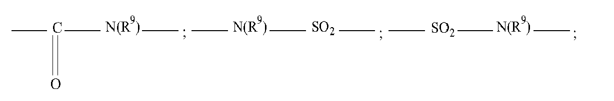 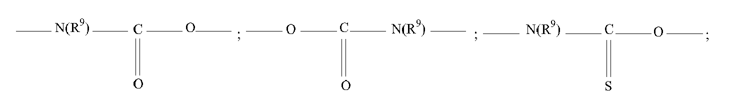 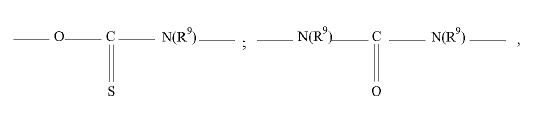 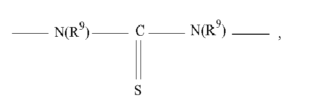 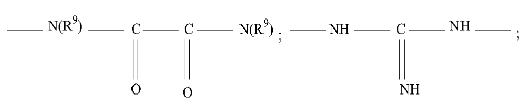 et 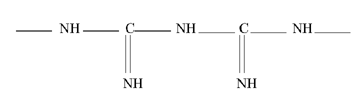 où R⁹ représente un atome d'hydrogène ou un groupe alkyle, linéaire ou ramifié, en C₁ à C₂₀, à condition qu'au moins 50 % des R⁹ du polymère représente un atome d'hydrogène et qu'au moins deux des groupes G du polymère soient un autre groupe que : 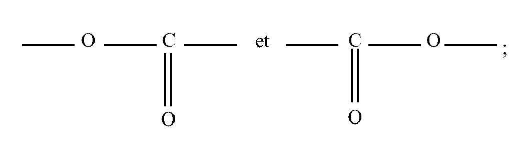
6) n est un nombre entier allant de 2 à 500, de préférence de 2 à 200, et m est un nombre entier allant de 1 à 1000, de préférence de 1 à 700 et mieux encore de 6 à 200.

9. Composition selon la revendication précédente, dans laquelle Y représente un groupe choisi parmi :
(a) les groupes alkylène linéaires en C₁ à C₂₀, de préférence en C₁ à C₁₀,
(b) les groupes alkylène ramifiés pouvant comporter des cycles et des insaturations non conjuguées, en C₃₀ à C₅₆,
(c) les groupes cycloalkylène en C₅-C₆,
(d) les groupes phénylène éventuellement substitués par un ou plusieurs groupes alkyle en C₁ à C₄₀,
(e) les groupes alkylène en C₁ à C₂₀, comportant de 1 à 5 groupes amides,
(f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆, et
(g) les chaînes polyorganosiloxane de formule : 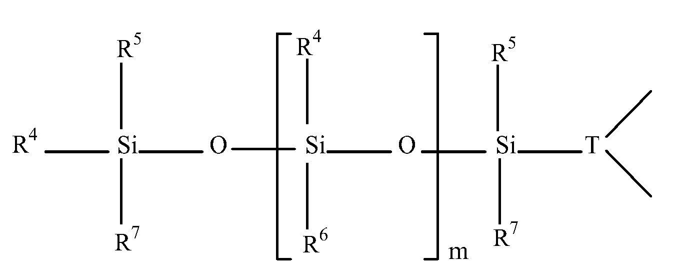 ou 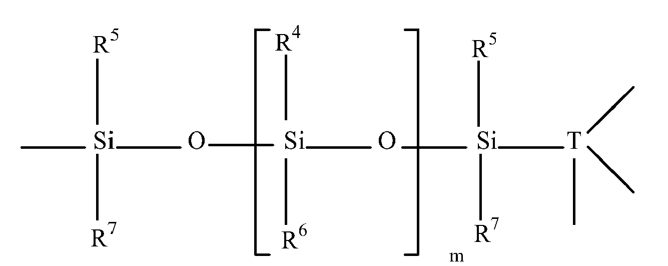
dans laquelle R⁴, R⁵, R⁶, R⁷, T et m sont tels que définis dans la revendication 8.

10. Composition selon l'une quelconque des revendications précédentes, dans laquelle le polymère comprend au moins un motif de formule (III) ou (IV) : 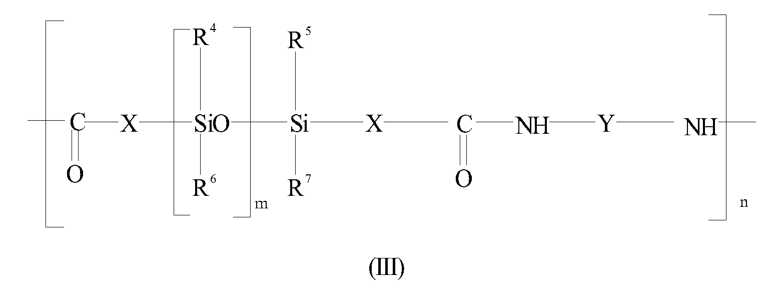 ou 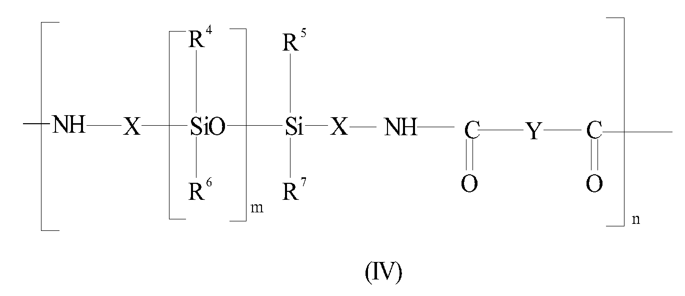 dans lesquelles R⁴, R⁵, R⁶, R⁷, X, Y, m et n sont tels que définis dans la revendication 8.

11. Composition selon la revendication précédente, dans laquelle X et/ou Y représentent un groupe alkylène contenant dans sa partie alkylène au moins l'un des éléments suivants :
1) 1 à 5 groupes amides, urée, uréthane, ou carbamate,
2) un groupe cycloalkyle en C₅ ou C₆, et
3) un groupe phénylène éventuellement substitué par 1 à 3 groupes alkyles identiques ou différents en C₁ à C₃,
et/ou substitué par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

12. Composition selon la revendication 10 ou 11, dans laquelle Y représente : 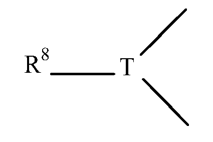 où R⁸ représente une chaîne polyorganosiloxane, et T représente un groupe de formule : 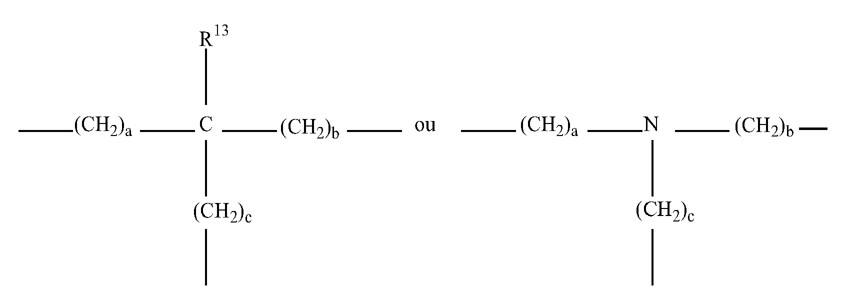 dans lesquelles a, b et c sont indépendamment des nombres entiers allant de 1 à 10, et R¹³ est un atome d'hydrogène ou un groupe tel que ceux définis pour R⁴, R⁵, R⁶ et R⁷, dans la revendication 8.

13. Composition selon l'une quelconque des revendications 10 à 12, dans laquelle R⁴, R⁵, R⁶ et R⁷ représentent, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

14. Composition selon l'une quelconque des revendications précédentes, dans laquelle le polymère comprend au moins un motif de formule (VII) : 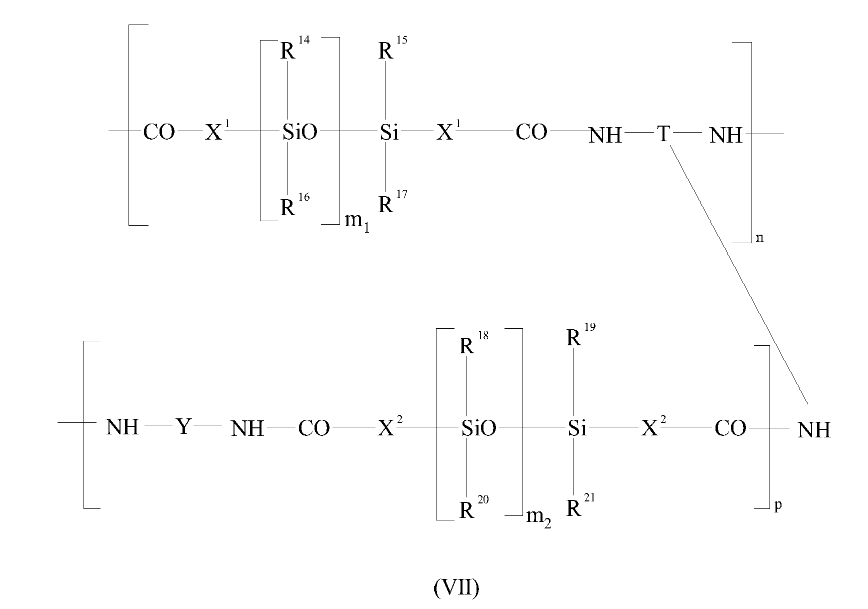 dans laquelle X¹ et X² qui sont identiques ou différents, ont la signification donnée pour X dans la revendication 8, n, Y et T sont tels que définis dans la revendication 8, R¹⁴ à R²¹ sont des groupes choisis dans le même groupe que les R⁴ à R⁷ de la revendication 8, m₁ et m₂ sont des nombres situés dans la gamme allant de 1 à 1 000, et p est un nombre entier allant de 2 à 500.

15. Composition selon la revendication précédente dans laquelle :
- p va de 1 à 25, mieux encore de 1 à 7,
- R¹⁴ à R²¹ sont des groupes méthyle,
- T répond à l'une des formules suivantes : 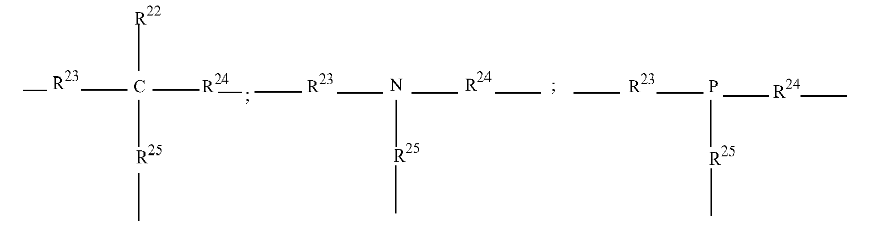 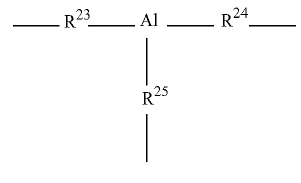
dans lesquelles R²² est un atome d'hydrogène ou un groupe choisi parmi les groupes définis pour R⁴ à R⁷, en revendication 8, et R²³ R²⁴ et R²⁵ sont indépendamment des groupes alkylène, linéaires ou ramifiés, de préférence encore, à la formule : 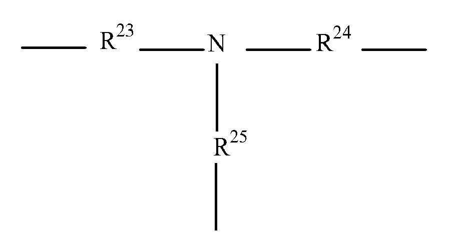 en particulier avec R²³, R²⁴ et R²⁵ représentant -CH₂-CH₂-,
- m₁ et m₂ vont de 15 à 500, et mieux encore de 15 à 45,
- X₁ et X₂ représentent -(CH₂)₁₀-, et
- Y représente -CH₂-.

16. Composition selon l'une quelconque des revendications précédentes, dans laquelle le polymère siliconé comprend au moins un motif répondant à la formule suivante (VIII) : 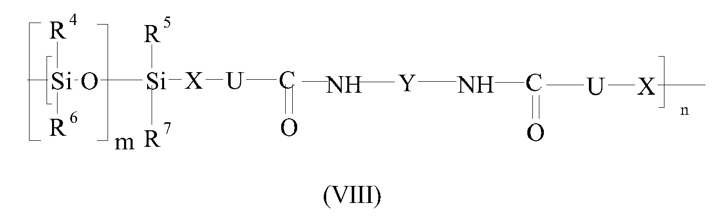 dans laquelle les R⁴, R⁵, R⁶, R⁷, X, Y, m et n ont les significations données pour la formule (I) dans la revendication 8
U représente -O- ou -NH-, afin que : 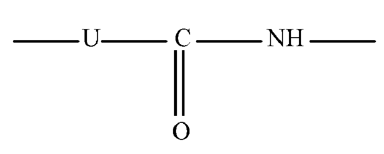 corresponde à un groupe uréthane ou urée, ou
Y représente un groupe cycloaliphatique ou aromatique en C₅ à C₁₂ pouvant être substitué par un groupe alkyle en C₁ à C₁₅ ou un groupe aryle en C₅ à C₁₀, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane, ou Y représente un radical alkylène en C₁ à C₄₀, linéaire ou ramifié, ou un radical cycloalkylène en C₄ à C₁₂, ou Y représente une séquence polyuréthane ou polyurée correspondant à la condensation de plusieurs molécules de diisocyanate avec une ou plusieurs molécules de couplage du type diol ou diamine, répondant à la formule (IX) : 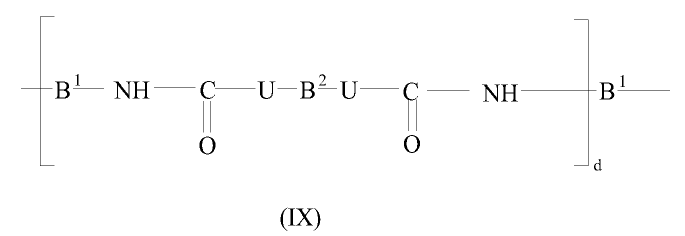 dans laquelle B¹ est un groupe choisi parmi les groupes donnés ci-dessus pour Y, U est -O- ou -NH-, et B² est choisi parmi :
• les groupes alkylène en C₁ à C₄₀, linéaires ou ramifiés,
• les groupes cycloalkylène en C₅ à C₁₂, éventuellement porteurs de substituants alkyle,
• les groupes phénylène pouvant éventuellement être porteurs de substituants alkyles en C₁ à C₃, et
• les groupes de formule : 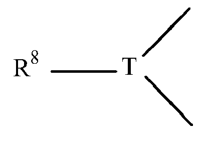
dans laquelle T est un radical trivalent hydrocarboné pouvant contenir un ou plusieurs hétéroatomes tels que l'oxygène, le soufre et l'azote et R⁸ est une chaîne polyorganosiloxane ou une chaîne alkyle en C₁ à C₅₀, linéaire ou ramifiée.

17. Composition selon l'une quelconque des revendications précédentes, **caractérisée en ce que** le polymère siliconé est choisi parmi les polymères comprenant au moins un motif répondant à la formule (II) : 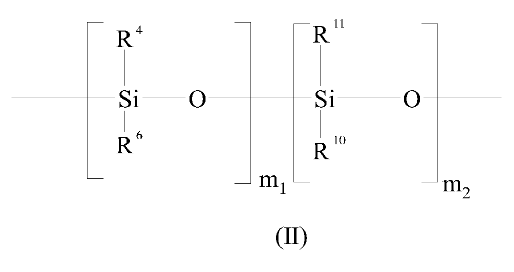 dans laquelle
R⁴ et R⁶, identiques ou différents, sont tels que définis ci-dessus pour la formule (I) en revendication 8,
R¹⁰ représente un groupe tel que défini ci-dessus pour R⁴ et R⁶, ou représente le groupe de formule -X-G-R¹² dans laquelle X et G sont tels que définis en revendication 8 et R¹² représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
R¹¹ représente le groupe de formule -X-G-R¹² ans laquelle X, G et R¹² sont tels que définis ci-dessus,
m₁ est un nombre entier allant de 1 à 998, et
m₂ est un nombre entier allant de 2 à 500.

18. Composition selon l'une quelconque des revendications précédentes, comprenant au moins un polymère bloc polydiméthylsiloxane de formule générale (I) telle que définie en revendication 8 et possédant un indice m de valeur supérieure à 50, en particulier supérieure à 75, mieux supérieure à 100 et notamment d'environ 120.

19. Composition selon l'une quelconque des revendications précédentes, comprenant au moins un premier polymère et au moins un second polymère comprenant chacun indépendamment au moins un motif de formule (III) telle que définie en revendication 10 dans laquelle :
- m varie de 50 à 600, en particulier de 60 à 400, notamment de 75 à 200 et plus particulièrement est de l'ordre de 120 pour le premier polymère et
- m va de 5 à 100, en particulier de 10 à 75 et plus particulièrement est de l'ordre de 15 pour le second polymère.

20. Composition selon la revendication précédente, dans laquelle le premier polymère a une masse moléculaire moyenne en poids comprise entre de 1 000 et 500 000 g/mol, notamment entre 10 000 et 300 000 g/mol.

21. Composition selon la revendication 19 ou 20, dans laquelle le deuxième polymère a de préférence une masse moléculaire moyenne en poids allant de 50 000 à 500 000 g/mol.

22. Composition selon l'une quelconque des revendications précédentes, dans laquelle le tensioactif phosphaté est choisi parmi :
(1) les composés de formule (XIV) : 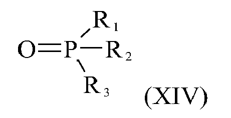 avec R₁, R₂ et R₃ identiques ou différents choisis parmi :
- un groupe OM avec M représentant un métal alcalin, tel que Na, Li, K,
- un groupe OR₄, dans lequel R₄ représente un groupe alkyle en C₅-C₄₀ linéaire, ramifié, cyclique ou aromatique,
- un groupement OH et
- un groupe oxyéthyléné (OCH₂CH₂)ₙ(OCH₂CHCH₃)ₘOR avec R représentant un atome hydrogène ou un groupe alkyle, linéaire ou ramifié, en C₁ à C₂₀ et n et m étant des nombres entiers, avec n variant de 1 à 50 et m variant de 0 à 50,
(2) les glycérophospholipides, et
(3) leurs mélanges.

23. Composition selon la revendication précédente dans laquelle R₁, R₂ et R₃ sont identiques.

24. Composition selon la revendication 22 ou 23 dans laquelle au moins un, notamment deux, voire l'ensemble des radicaux R₁, R₂ et/ou R₃ figure(nt) un groupement alkyle linéaire ou ramifié, en C₁₀ à C₃₀, voire en C₁₈.

25. Composition selon l'une quelconque des revendications 22 à 24, dans laquelle R est un radical alkyle en C₁₂ à C₁₅.

26. Composition selon l'une quelconque des revendications 22 à 25, dans laquelle n = 10 et/ou m = 0.

27. Composition selon l'une quelconque des revendications 22 à 26, dans laquelle le tensioactif phosphaté comprend du phosphate de trioléyle, du tri-C₁₂₋₁₅ pareth-10 phosphate, ou du cetyl phosphate de potassium.

28. Composition selon les revendications 22 à 27, dans laquelle le tensioactif phosphaté comprend du phosphate de trioléyle.

29. Composition selon l'une quelconque des revendications 22 à 28, dans laquelle le glycérophospholipide est défini selon la formule générale (XV) suivante : 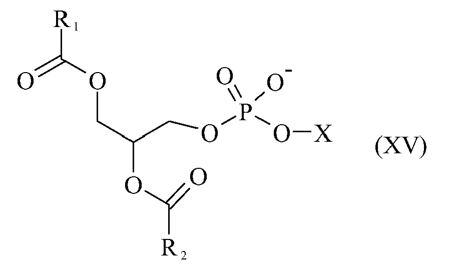 dans laquelle :
- R₁ et R₂ représentent, indépendamment l'un de l'autre, un acide gras comprenant de 4 à 24 atomes de carbone, saturé ou insaturé, le cas échéant ramifié, et susceptible d'être substitué par une ou plusieurs fonctions hydroxy et/ou amines, et
- X représente un substituant de formule générale R₃R₄R₅N⁺-CH(R₆)-CH₂- dans lequel R₃, R₄, R₅ et R₆ représentent, indépendamment l'un de l'autre, un hydrogène, des groupes alkyles comprenant de 1 à 6 atomes de carbone, et/ou une fonction carboxy.

30. Composition selon la revendication précédente, dans laquelle R₁ et R₂, indépendamment l'un de l'autre, sont choisis parmi l'acide butyrique, l'acide caproïque, l'acide caprylique, l'acide caprique, l'acide caproléïque, l'acide laurique, l'acide lauroléïque, l'acide myristique, l'acide myristoléique, l'acide palmitique, l'acide palmitoléique, l'acide stéarique, l'acide oléique, l'acide linoléique, l'acide linolénique, l'acide arachidique, l'acide isostéarique, l'acide dihydroxystéarique et l'acide ricinoléique.

31. Composition selon la revendication 29 ou 30, dans laquelle X est choisi parmi la choline, la sérine et/ou l'éthanolamine.

32. Composition selon l'une quelconque des revendications 29 à 31, dans laquelle le glycérophospholipide comprend de la phosphatidylcholine, de la phosphatidyléthanolamine et/ou de la phosphatidylsérine.

33. Composition selon la revendication précédente, dans laquelle le glycérophospholipide est de la lécithine.

34. Composition selon l'une quelconque des revendications 22 à 33, dans laquelle le glycérophospholipide est un glycérophospholipide non hydrogéné.

35. Composition selon l'une quelconque des revendications précédentes, comprenant un tensioactif phosphaté non ionique.

36. Composition selon l'une quelconque des revendications précédentes, comprenant de 0,1 et 30 % notamment entre 0,1 et 10 % voire entre 0,5 et 5 % en poids de tensioactif phosphaté par rapport au poids total de la composition.

37. Composition selon l'une quelconque des revendications précédentes, comprenant, à titre de tensioactif phosphaté, du phosphate de trioleyle et/ou de la lécithine et un polyamide/polydiméthylsiloxane et/ou un polymère siliconé comprenant au moins un motif de formule (III) telle que définie en revendication 8.

38. Composition selon l'une quelconque des revendications précédentes, comprenant une phase grasse.

39. Composition selon l'une quelconque des revendications précédentes, comprenant une huile siliconée.

40. Composition selon l'une quelconque des revendications précédentes, comprenant une huile hydrocarbonée.

41. Composition selon l'une quelconque des revendications précédentes, comprenant moins de 10 %, voire moins de 4 % d'eau, en particulier étant anhydre.

42. Composition selon l'une quelconque des revendications précédentes, comprenant en outre un actif choisi parmi :
- les agents dermorelaxants,
- les agents stimulant la synthèse de macromolécules dermiques ou épidermiques et/ou empêchant leur dégradation,
- les agents anti-glycation,
- les agents anti-irritants,
- les agents hydratants,
- les agents desquamants
- les agents dépigmentant, anti-pigmentant ou pro-pigmentant,
- les inhibiteurs de la NO-synthase,
- les agents stimulant la prolifération des fibroblastes ou des kératinocyles et/ou la différentiation des kératinocytes,
- les agents anti-pollution ou anti-radicalaire,
- les agents apaisants,
- les agents agissant sur la microcirculation,
- les agents agissant sur le métabolisme énergétique des cellules,
- les agents cicatrisants, et
- leurs mélanges.

43. Composition selon la revendication précédente, comprenant de la glycérine.

44. Composition selon l'une quelconque des revendications précédentes, comprenant un polymère filmogène.

45. Composition selon l'une quelconque des revendications précédentes, comprenant une matière colorante.

46. Procédé de maquillage des lèvres, dans lequel on applique sur les lèvres, une composition telle que définie selon l'une quelconque des revendications précédentes.

47. Utilisation d'un polymère siliconé tel que défini en revendications 2 à 21 en association avec un tensioactif phosphaté tel que défini en revendications 22 à 34 pour préparer une composition cosmétique pour les lèvres et apte à procurer un film de brillance améliorée.

48. Utilisation d'un polymère siliconé tel que défini en revendications 2 à 21 en association avec un tensioactif phosphaté tel que défini en revendications 22 à 34 pour assurer une dispersion homogène et stabilisée de particules solides telles que par exemple des pigments dans une composition cosmétique pour les lèvres.
